# EUROPÄISCHE PATENTSCHRIFT

(11) **EP 0 290 570 B1**
(45) Veröffentlichungstag und Bekanntmachung des Hinweises auf die Patenterteilung: **23.09.1992**
(21) Anmeldenummer: 87907946.5
(22) Anmeldetag: 30.10.1987
(51) Int. Cl.: C07D 239/26, C07D 213/30, C07C 255/01, C09K 19/12, C09K 19/34

(54) **CHIRALE ODER ACHIRALE RINGVERBINDUNGEN**
CHIRAL OR ACHIRAL CYCLIC COMPOUNDS
COMPOSES CYCLIQUES CHIRAUX OU ACHIRAUX

(30) Priorität: 07.11.1986 DE 3638026
(43) Veröffentlichungstag der Anmeldung: 17.11.1988
(73) Patentinhaber: MERCK PATENT GmbH, D-64271 Darmstadt (DE)
(72) Erfinder: WÄCHTLER, Andreas, D-6103 Griesheim (DE); KRAUSE, Joachim, D-6110 Dieburg (DE); PAULUTH, Detlef, D-6100 Darmstadt (DE); GEELHAAR, Thomas, D-6500 Mainz (DE)
(86) Internationale Anmeldenummer: EP8700643
(87) Internationale Veröffentlichungsnummer: WO8803530

(56) Entgegenhaltungen:
- EP-A- 0 191 860
- WO-A-86/06373

## Beschreibung

Die Erfindung betrifft chirale oder achirale Ringverbindungen der Formel 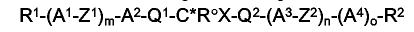 worin
R¹ und R² jeweils unabhängig voneinander eine Alkyl- oder Perfluoralkyl-Gruppe mit jeweils 1-12 C-Atomen, worin auch eine oder zwei nicht benachbarte CH₂- bzw. CF₂-Gruppen durch O-Atome und/oder-CO-Gruppen und/oder -CO-O-Gruppen und/oder -CH=CH-Gruppen und/oder -CHHalogen- und/oder -CHCN-Gruppen und/oder -O-CO-CHHalogen- und/oder -CO-O- CHCN-Gruppen ersetzt sein können,
eine der Gruppen R¹ und R² auch H, F, Cl, Br oder CN, R¹ auch -Q¹-C*R°X-Q²-R², wobei Q^{l}, R°, X, Q² und R² die angegebenen Bedeutungen haben,
A¹, A²A³ und A⁴ jeweils unabhängig voneinander unsubstituiertes oder durch ein oder zwei F- und/oder CI-Atome und/oder CH₃-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte CH₂-Gruppen durch O-Atome und/ oder S-Atome ersetzt sein können, Piperidin-1,4-diyl, 1,4-Bicyclo(2,2,2)oc- tylen-, 1,3,4-Thiadiazol-2,5-diyl, Naphthalin-2,6-diyl-, Decahydronaphthalin-2,6-diyl-oder 1,2,3,4-Tetrahydronaphthalin -2,6-diyl-,
Z¹ und Z² jeweils -CO-O-, -O-CO-, -CH₂CH₂-, -OCH₂-, -CH₂0-, -C≡C- oder eine Einfachbindung,
X Halogen, CN oder CH₃,
R° eine von X (und im Falle n= o= O auch von R²) verschiedene Alkylgruppe mit 1 bis 10 C-Atomen,
C^{*} ein mit vier verschiedenen Substituenten verknüpftes Kohlenstoffatom,
m und n jeweils unabhängig voneinander 0, 1, 2 oder 3,
o 0 oder 1,
(m + n + o) 1, 2, 3 oder 4, und
Q¹ und Q² jeweils unabhängig voneinander Alkylen mit 1 bis 4 C-Atomen, worin auch eine CH₂-Gruppe durch -O-, -S-, -CO-, -O-CO-, -CO-O-, -S-CO-, -CO-S-, -CH=CH-COO-, -CH=CH-, -CHHalogen und/ oder - CHCN- ersetzt sein kann, oder eine Einfachbindung

bedeuten.

Die Verbindungen der Formel können wie ähnliche in DE-OS 35 15 373 beschriebene Verbindungen als Komponenten chiraler getilteter smektischer flüssigkristalliner Phasen verwendet werden.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44, (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltetwerden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc^{*}) ist deren relativ hohe optische Anisotropie, die durch relativ hohe Viskositätswerte bedingten nicht ausreichend kurzen Schaltzeiten, sowie, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/ Florida, April/Mai 1985, Vortrag 8.3).

Es wurde nun gefunden, daß die Verwendung von Verbindungen der Formel 1 als Komponenten chiraler getilteter smektischer Mischungen die erwähnten Nachteile wesentlich vermindern kann. Die Verbindungen der Formel sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc^{*-}Phasenbereichen, negativer oder auch positiver dielektrischer Anisotropie, niedriger optischer Anisotropie, günstiger Pitchhöhe, niedriger Viskosität und für derartige Phasen hohen Werten für die spontane Polarisation und sehr kurzen Schaltzeiten herstellbar. P ist die spontane Polarisation in nC/cm².

Mit der Bereitstellung der Verbindungen der Formel wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formel eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und weisen günstige Werte der optischen Anisotropie auf. Teilweise zeigen die Verbindungen der Formel flüssigkristalline Mesophasen in einem für die elektrooptische Verwendung günstig gelegenen Temperaturbereich, es können jedoch auch isotrope oder monotrop flüssigkristalline Verbindungen der Formel als Komponenten chiraler getilteter smektischer Phasen vorteilhaft eingesetzt werden. Chemisch, thermisch und gegen Licht sind sie sehr stabil.

Gegenstand der Erfindung sind somit die Verbindungen der Formel 1 sowie die Verwendung der Verbindungen der Formel als Komponenten flüssigkristalliner Phasen.

Gegenstand der Erfindung sind auch chirale getiltete smektische flüssigkristalline Phasen mit einem Gehalt an mindestens einer Verbindung der Formel mit mindestens einem mit vier verschiedenen Substituenten verknüpften Kohlenstoffatom.

Gegenstand der Erfindung sind ferner solche Phasen mit einem Gehalt an mindestens einer Verbindung der Formel I sowie Flüssigkristallanzeigeelemente, insbesondere elektrooptische Anzeigeelemente, die derartige Phasen enthalten.

Der Einfachheit halber bedeuten im folgenden Ph eine 1,4-Phenylengruppe, worin auch eine oder zwei CH-Gruppen durch N ersetzt sein können, Cy eine 1,4-Cyclohexylengruppe , worin auch eine oder zwei nicht benachbarte CH₂-Gruppen durch O-Atome ersetzt sein können und Bi eine Bicyclo(2,2,2)octylengruppe.

Vor- und nachstehend haben R¹, R², m, o, n, A¹, A², A³, A⁴, Q¹, Q², X, R°, Z¹ und Z² die angegebene Bedeutung, sofern nicht ausdrücklich etwas anderes vermerkt ist.

Die Verbindungen der Formel umfassen dementsprechend insbesondere Verbindungen der Teilformeln la und Ib (mit zwei Rinaen) 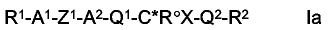 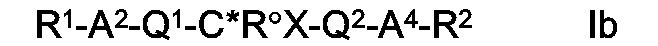 und Ic bis le (mit drei Ringen): 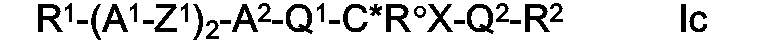 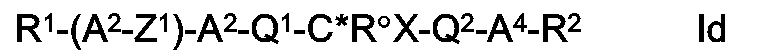 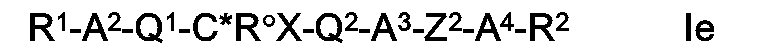 und If bis Ih (mit vier Ringen):

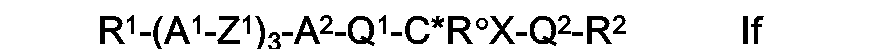

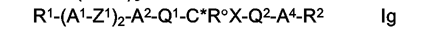

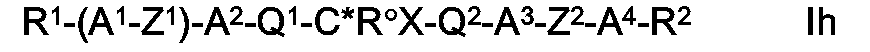

Darunter sind diejenigen der Formeln la, Ib und Ic besonders bevorzugt.

Die bevorzuaten Verbindunaen der Formel la umfassen solche der Teilformeln la1 bis Ia4:

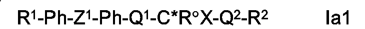

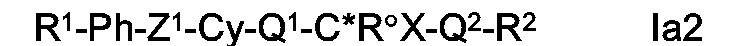

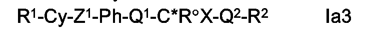

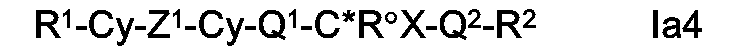

Darunter sind diejenigen der Teilformeln lal besonders bevorzugt.

Die bevorzuaten Verbindunaen der Formel Ib umfassen solche der Teilformeln Ibl bis Ib4:

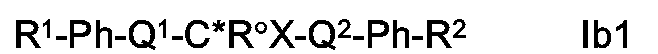

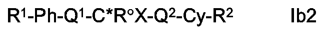

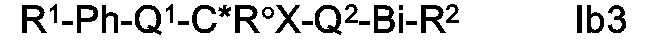

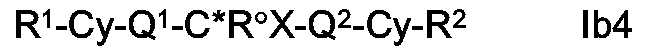

Darunter sind diejenigen der Teilformeln Ibl besonders bevorzugt.

Die bevorzugten Verbindungen der Formel Ic umfassen solche der Teilformeln Ic1 bis Ic8:

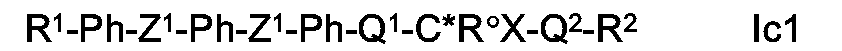

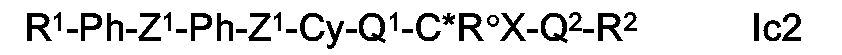

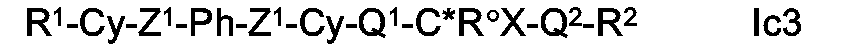

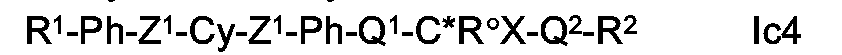

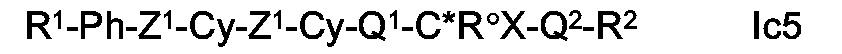

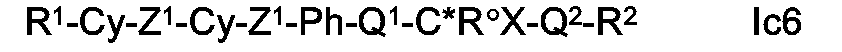

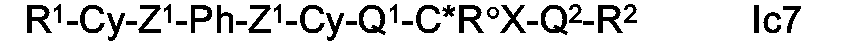

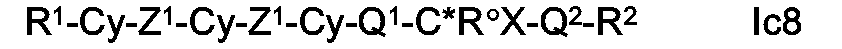

Besonders bevorzugt sind Verbindungen der Formel I'
R¹-(A¹-Z¹)ₘ-A²-Q¹-C*R°X-Q²-R²

worin m 1 oder 2 bedeutet und R¹, R², A¹, A², Z¹, Q¹, Q², R° und X die oben angegebene Bedeutung haben. R° ist eine von X und Q²⁻R² verschiedene Alkylgruppe mit vorzugsweise 1 bis 5 C-Atomen. Besonders bevorzugt sind Methyl und Ethyl, insbesondere Methyl. R² ist vorzugsweise eine Alkylgruppe mit 2 bis 10, insbesondere mit 2 bis 6, C-Atomen. Q¹ und Q² bedeuten vorzugsweise jeweils unabhängig voneinander-O-CO-(wobei das Carbonylkohlenstoffatom mit dem asymmetrischen C-Atom C^{*} verknüpft ist), -0-CH₂-(wobei die Methylengruppe mit dem asymmetrischen C-Atom C^{*} verknüpft ist), -CH₂CH₂-, -CH₂- oder eine Einfachbindung (-). Besonders bevorzugte Kombinationen von Q¹ und Q² sind in der folgenden Tabelle angegeben:

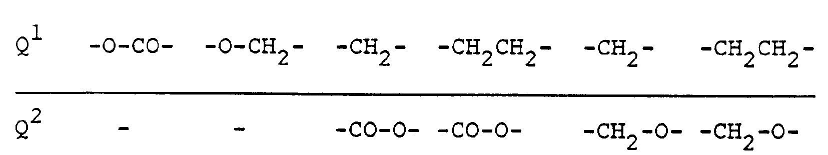

In den bevorzugten Verbindungen der vor- und nachstehenden Formeln können die Alkylreste, in denen auch eine CH₂-Gruppe (Alkoxy bzw. Oxaalkyl) durch ein 0-Atom ersetzt sein kann, geradkettig oder verzweigt sein. Vorzugsweise haben sie 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2-(= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

A¹, A², A³ und A⁴ sind bevorzugt Cy oder Ph. In den Verbindungen der vor- und nachstehenden Formeln bedeutet Ph vorzugsweise eine 1,4-Phenylen- (Phe), eine Pyrimidin-2,5-diyl- (Pyr), eine Pyridin-2,5-diyl- (Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind. Cy bedeutet vorzugsweise eine 1,4-Cyclohexylengruppe. Insbesondere bevorzugt sind jedoch Verbindungen der Formel I, worin eine der Gruppen A², A³ und A⁴ eine in 1-oder 4-Position durch CN substituierte 1,4-Cyclohexylengruppe bedeutet und die Nitrilgruppe sich in axialer Position befindet, d.h. die Gruppe A², A³ bzw. A⁴ die folgende Konfiguration aufweist:

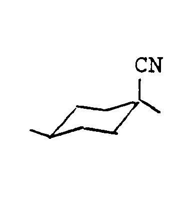

Besonders bevorzugt sind Verbindungen der Formel und der vorstehenden Teilformeln, die eine Gruppierung -Ph-Ph- enthalten. -Ph-Ph- ist vorzugsweise -Phe-Phe-, Phe-Pyr oder Phe-Pyn. Besonders bevorzugt sind die Gruppen 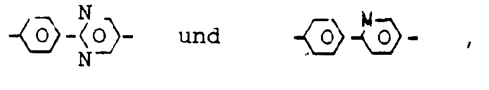 sowie ferner unsubstituiertes oder ein- oder mehrfach durch Fluor substituiertes 4,4'-Biphenylyl.

Z¹ und Z² sind bevorzugt Einfachbindungen, in zweiter Linie bevorzugt -O-CO-, -CO-0-, -C≡C- oder - CH₂CH₂-Gruppen.

Besonders bevorzugt für Z¹ ist -CO-O, -O-CO-, -C≡C- oder -CH₂CH₂-, insbesondere die -CH₂CH₂-und die -C≡C-Gruppe.

X bedeutet in den Verbindungen der vor- und nachstehenden Formeln Halogen, CN oder CH₃, vorzugsweise F, Cl, CH₃ oder CN. Besonders bevorzugt sind F und CN.

R° ist eine von X verschiedene, vorzugsweise geradkettige Alkylgruppe mit vorzugsweise bis zu 4 C-Atomen. Besonders bevorzugt wird Methyl und Ethyl, insbesondere Methyl.

Die bevorzugte Bedeutung von Q¹ und Q² ist Alkylen mit 1 bis 2 C-Atomen, -O-, -O-CO-, -CO-O- und eine Einfachbindung. Ferner bevorzugte Bedeutungen von Q¹ bzw. Q² sind -CH₂0- und -O-CH_{Z}-.

Verbindungen der vor- und nachstehenden Formeln mit verzweigten Flügelgruppen R¹ bzw. R² können von Bedeutung sein. Verzweigte Gruppen dieser Art enthalten in der Regel nicht mehr als zwei Kettenverzweigungen. R¹ ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Der Rest R¹ und insbesondere auch der Rest R² kann auch ein optisch aktiver organischer Rest mit einem asymmetrischen Kohlenstoffatom sein. Vorzugsweise ist dann das asymmetrische Kohlenstoffatom mit zwei unterschiedlich substituierten C-Atomen, einem H-Atom und einem Substituenten ausgewählt aus der Gruppe Halogen (insbesondere F, CI oder Br), Alkyl oder Alkoxy mit jeweils 1 bis 5 C-Atomen und CN verknüpft. Der optisch aktive organische Rest hat vorzugsweise die Formel, 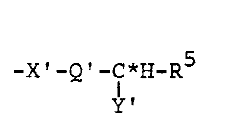 worin X' -CO-O-, -O-CO-, -O-CO-O-, -CO-, -O-, -S-, -CH=CH-, -CH=CH-COO-odereine Einfachbindung,
Q' Alkylen mit 1 bis 5 C-Atomen, worin auch eine nicht mit X' verknüpfte CH₂-Gruppe durch -O-, -CO-, -O-CO-, -CO-O- oder -CH=CH- ersetzt sein kann, oder eine Einfachbindung,
Y' CN, Halogen, Methyl oder Methoxy, und
R⁵ eine von Y verschiedene Alkylgruppe mit 1 bis 15 C-Atomen, worin auch eine oder zwei nicht benachbarte CH₂-Gruppen durch -O-, -CO-, -O-CO-, -CO-O- und/oder -CH=CH- ersetzt sein können, bedeutet.
X' ist vorzugsweise -CO-O-, -O-CO-, -CH=CH-COO- (trans) oder eine Einfachbindung. Besonders bevorzugt sind -CO-O-/-O-CO- oder eine Einfachbindung.
Q' istvorzugsweise -CH₂-,-CH₂CH₂-,-CH₂CH₂CH₂- oder eine Einfachbindung, insbesondere bevorzugt eine Einfachbindung.
Y' ist vorzugsweise CH₃, -CN, F oder Cl, insbesondere bevorzugt CN oder F.
R⁵ ist vorzugsweise geradkettiges oder verzweigtes Alkyl mit 1 bis 10, insbesondere mit 1 bis 7, C-Atomen.

Unter den Verbindungen der Formel sind diejenigen bevorzugt, in denen X' und Y' nicht gleichzeitig Methyl bedeuten.

Unter den Verbindungen der Formel sowie la bis Ih sind diejenigen bevorzugt, in denen mindestens einer der darin enthaltenen Reste eine der angegebenen bevorzugten Bedeutungen hat.

In den Verbindungen der Formel sowie in den vor- und nachstehenden Teilformeln ist -(A¹-Z¹)ₘ-A²- vorzugsweise eine Gruppe der folgenden Formeln 1 bis 38 oder deren Spiegelbild:

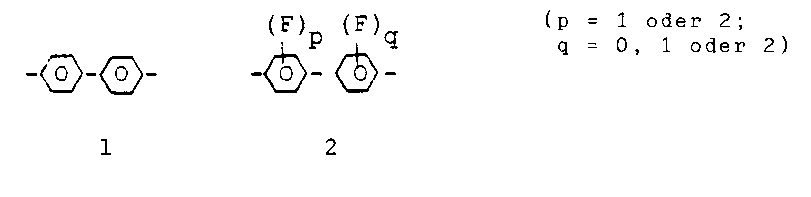

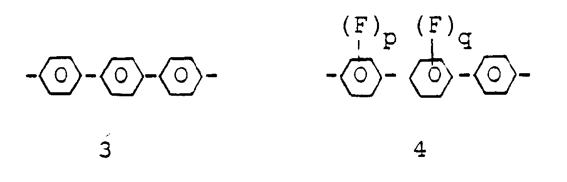

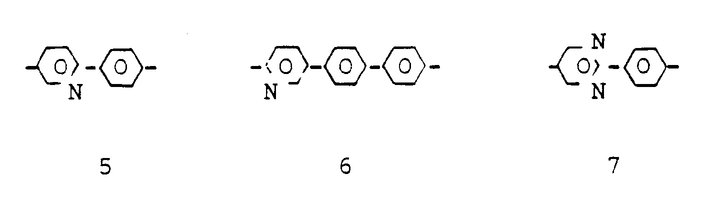

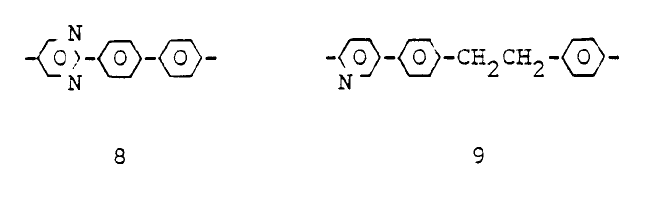

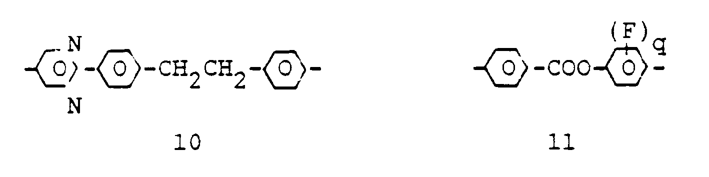

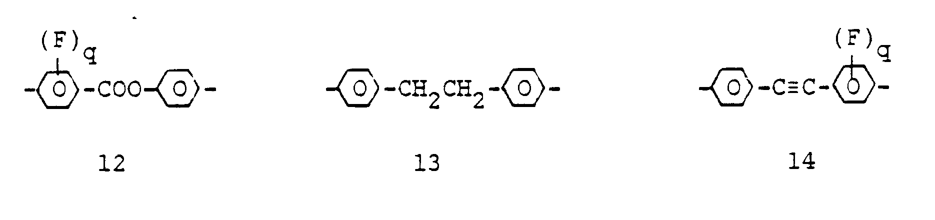

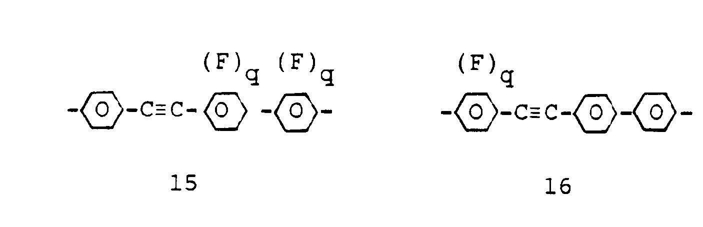

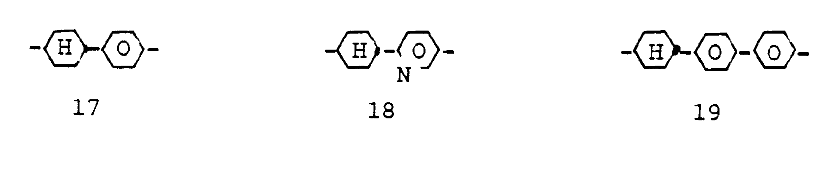

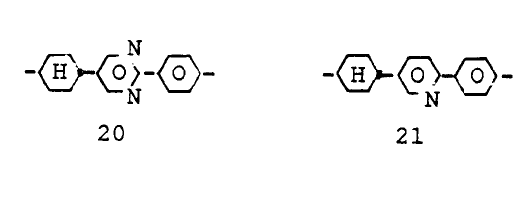

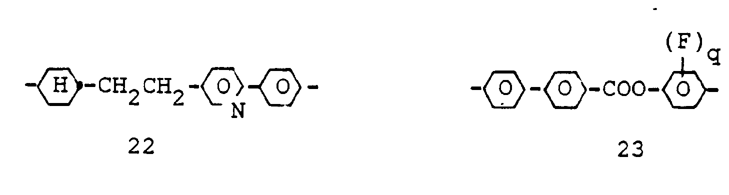

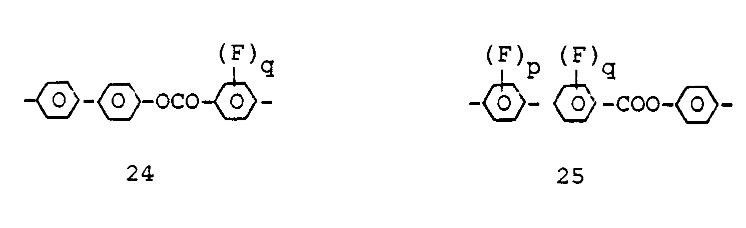

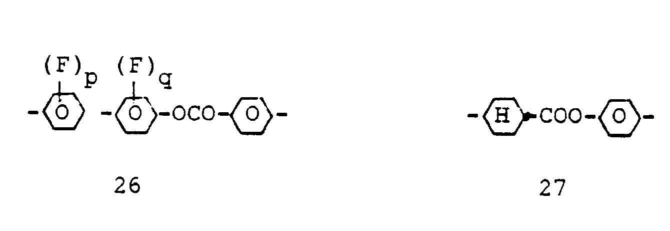

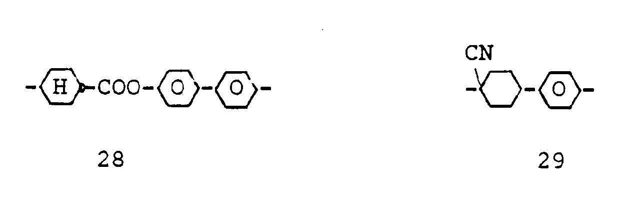

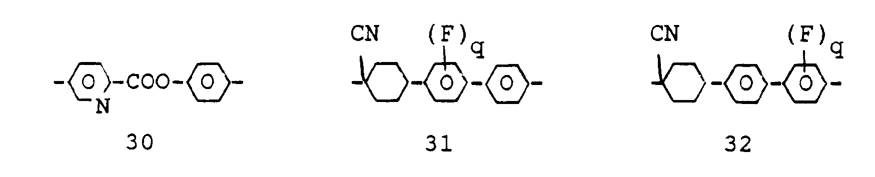

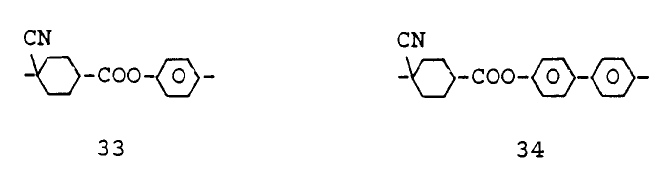

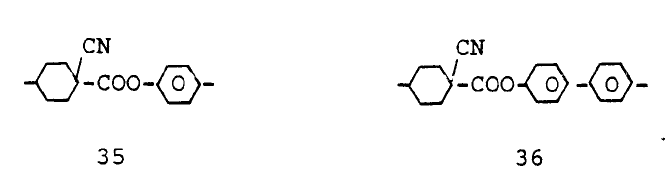

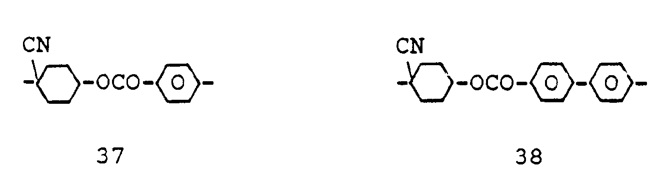

Gruppen der Formeln 1, 5, 7, 9, 10, 11, 12, 13 und 14, insbesondere diejenigen der Formeln 5 und 7, sind besonders bevorzugt.

Besonders bevorzugt sind ferner Phenylpyrimidinderivate der Formel A 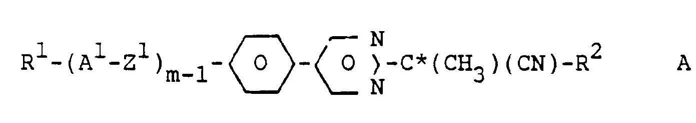 worin R¹, A¹, Z¹, m und R² die bei Formel angegebene Bedeutung haben. Verbindungen der Formel A sind erhältlich durch Umsetzung von geeigneten Methylcyanessigsäureethylestern mit gasförmigem Chlorwasserstoff in Ethanol,

Umsetzung des erhaltenen Iminoethers mit Ammoniak in das entsprechende Amidinhydrochlorid, wobei gleichzeitig die Estergruppe in die Amidgruppe übergeht, und anschließende übliche Kondensation mit p-substituierten 3-(Dimethylamino)-acroleinen bzw. mit entsprechenden Phenylmalondialdehyden (G.M. Coppola et al., J. Heterocycl. Chem. 11, 51 (1974)):

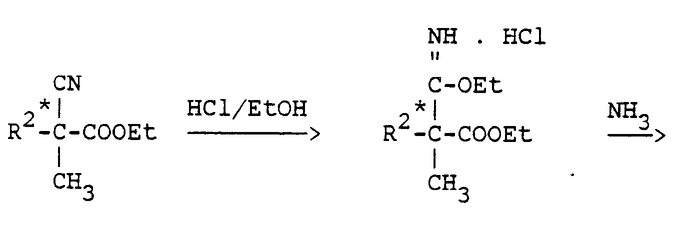

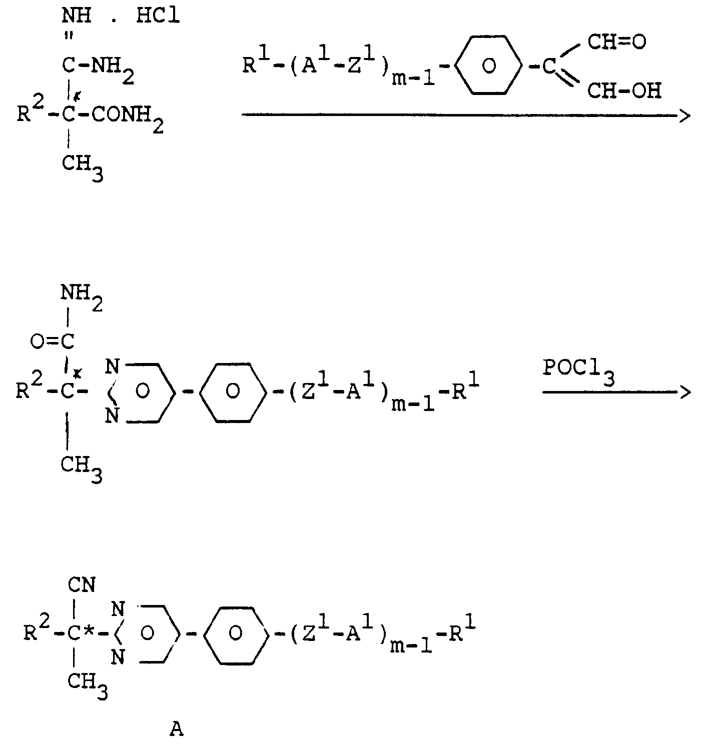

Einige weitere bevorzugte kleinere Gruppe von Verbindungen der Formel sind diejenigen der Teilformeln B, C, D, E, F, und G: 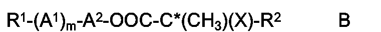 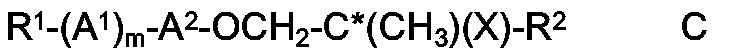 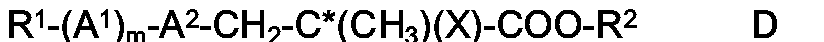 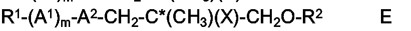 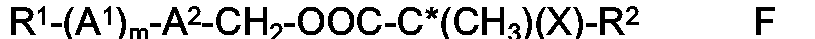 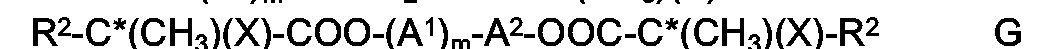 worin R¹, R², A¹ und A² die bei Formel angegebene Bedeutung haben. m ist vorzugsweise 1 oder 2. -(A¹)ₘ-A²-bedeutet vorzugsweise 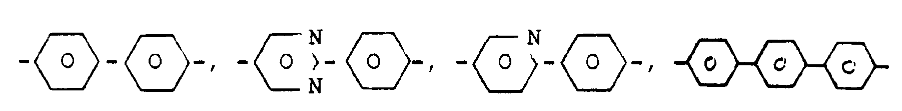 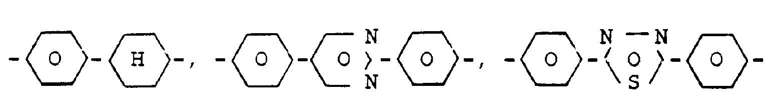 oder 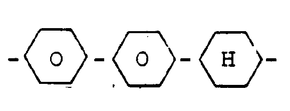 oder ein Spiegelbild dieser Formeln. R¹ ist vorzugsweise geradkettiges Alkyl oderAlkoxy mit 5 bis 12 C-Atomen. R² ist vorzugsweise geradkettiges oder verzweigtes Alkyl mit 1 bis 5 C-Atomen.

Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Die Verbindungen der Formel werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel umsetzt.

So können die Verbindungen der Formel oder zu deren Herstellung geeignete Vorstufen hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise -CH=CH-gruppen in Betracht, ferner z.B. freie oderveresterte Hydroxygruppen, aromatisch gebundene Halogenatome oder Carbonylgruppen. Bevorzugte Ausgangsstoffe für die Reduktion entsprechen der Formel I, können aber an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. Pt0₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAIH₄ reduktiv entfernt werden, insbesondere p-Toluol-sulfonyloxymethylgruppen zu Methylgruppen reduziert werden, zweckmäßig in einem inerten Lösungsmittel wie Di- ethylether oder THF bei Temperaturen zwischen etwa 0 und 100°. Doppelbindungen können (auch in Gegenwart von CN-Gruppen!) mit NaBH₄ oder Tributylzinnhydrid in Methanol hydriert werden; so entstehen z.B. aus 1-Cyancyclohexenderivaten die entsprechenden Cyclohexanderivate.

Ester der Formel können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführtwerden. Die Reaktionstemperatur liegt gewöhnlich zwischen -50° und +250_{°}, vorzugsweise zwischen -20° und +80_{°}. Bei diesen Temperaturen sind die Veresterungsreaktionen in der Regel nach 15 Minuten bis 48 Stunden beendet.

Im einzelnen hängen die Reaktionsbedinungen fürdie Veresterung weitgehend von der Naturderverwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. - phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20_{°}.

Dioxanderivate bzw. Dithianderivate der Formel werden zweckmäßig durch Reaktion eines entsprechenden Aldehyds (odereines seiner reaktionsfähigen Derivate) mit einem entsprechenden 1,3-Dioi bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Ether der Formel sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂C0₃ oder K₂C0₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen der Formel oder zur Herstellung von geeigneten Vorstufen können auch entsprechende Chlor- oder Bromverbindungen der Formel oder geeignete Vorstufen mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20° und 200°.

Die optisch aktiven Verbindungen der Formel erhält man durch den Einsatz entsprechender optisch aktiver Ausgangsmaterialien und/oder durch Trennung der optischen Antipoden mittels Chromatographie nach bekannten Methoden.

Ester der Formel worin einer der Reste Q¹ und Q² -CO-O- bedeutet und das Carbonylkohlenstoffatom direkt mit dem asymmetrischen C-Atom C^{*} verknüpft ist, können leicht aus den entsprechenden optisch aktiven disubstituierten Cyanessigsäuren durch Veresterung mit mesogenen Alkoholen oder Phenolen, z.B. der Formel R¹-(A¹-Z¹)ₘ-A²-OH oder R²-(A⁴)ₒ-(Z²-A³)ₙ-OH bzw. mit Alkoholen der Formel R²⁻OH hergestelltwerden. Die Veresterung kann nach einer der vorstehend genannten Methoden durchgeführt werden. Vorzugsweise bedient man sich jedoch der in den Beispielen angegebenen DCC-Methode.

Disubstituierte optisch aktive Cyanessigsäuren sind seit langem bekannt (E. Fischer, et al., Ber.Dtsch. Chem.Ges. 42, 2961 (1909); E.C. Kleiderer, H.A. Shonle, JACS 56,1772 (1934); J. Kenyon, W.A. Ross, J. Chem.Soc. (London) 1951, 3407).

Vor allem im Zusammenhang mit der Synthese optisch aktiver Barbiturate ist in jüngerer Zeit ausführlich über die Racematspaltung optisch aktiver Cyanessigsäuren mit Basen wie z.B. Brucin, Chinin, Kodein oder bevorzugt 1S.2S(+) bzw. 1 R.2R(-)-1-[4-Nitrophenyl]-2-aminopropandiol-(1.3) bzw. 1R.2R-2-Amino-1-phenyl- propandiol-(1,3) berichtet worden (J. Knabe, W. Rummel, H.P. Büch, N. Franz, Arzneim.-Forsch./Drug Res. 28 (11) 1048 (1978) et lit. cit). Insbesondere sei auf die Synthese und Racematspaltung von Ethylhexylcyanessigsäure (J. Knabe, H. Junginger, Pharmazie 27, 443 (1972)) und Methylethylcyanessigsäure (J. Knabe, N. Franz, Arch.Pharm. 309, 173 (1976)) hingewiesen. Die hier benutzten disubstituierten Cyanessigsäuren können analog durch Verseifung der entsprechenden Ethyl- oder Methylester und Racematspaltung hergestellt werden.

Die dazu benötigten disubstituierten Cyanessigsäureethylester sind entweder durch Knoevenagel-Kondensation entsprechender Aldehyde mit Cyanessigsäureethylester, Hydrierung der Vinylverbindung und Alkylierung oder aber im Fall Ro=Me auch durch Alkylierung von Methylcyanessigsäureethylester (C. de Hoffmann, E. Barbier, Bl.Soc. Chim.Belge 45, 565 (1936) leicht zugänglich.

Ether der Formel I, worin einer der Reste Q¹ und Q² -CH₂-O-bedeutet und die Methylengruppe direkt mit dem asymmetrischen C-Atom C^{*} verknüpft ist, können ausgehend von den vorstehend beschriebenen optisch aktiven disubstituierten Cyanessigsäuren hergestellt werden.

Hierzu werden z.B. gemäß folgendem Schema die optisch aktiven Cyanessigsäuren mit entsprechenden Thiolen in Gegenwart von Phenyldichlorophosphat und Pyridin nach H.J. Liu, S.I. Sabesan, Can.J.Chem. 58, 2645 (1980) in die entsprechenden Thiolester überführt und anschließend mit NaBH₄ nach H.J. Liu, H. Wyun, Tetrahedron Letters 23, 3151 (1982) zu den a,a-disubstituierten optisch aktiven β-Hydroxypropionitrilen reduziert werden. Diese β-Hydroxypropionitrile werden mit geeigneten Säuren, z.B. der Formel R¹-(A¹-Z¹)ₘ-A²-COOH oder R²-(A⁴)ₒ-(Z²-A³)ₙ-COOH oder mit Säuren der Formel R²⁻COOH, z.B. nach der DCC-Methode verestert.

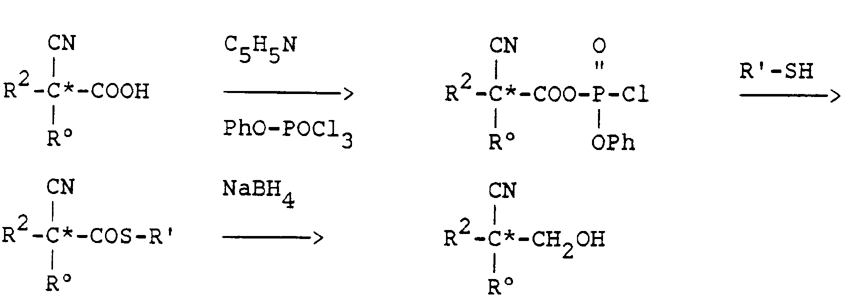

Beispiele für geeignete mesogene Säuren sind im folgenden aufgeführt:

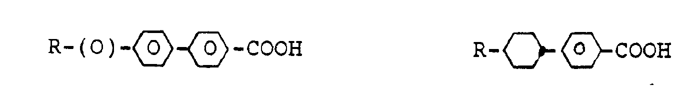

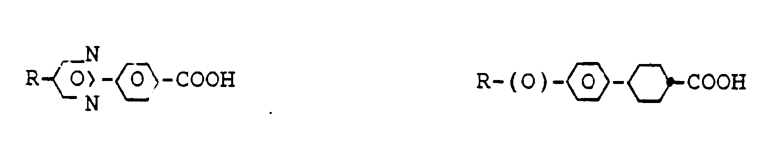

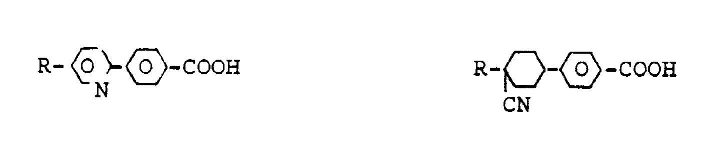

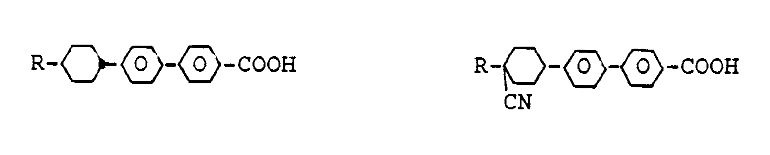

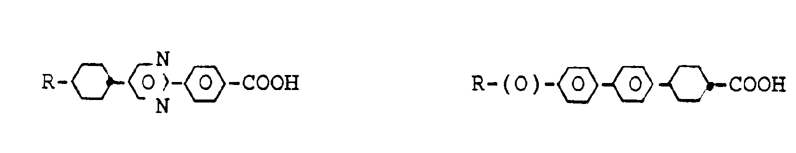

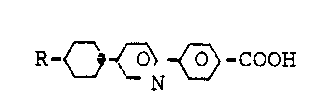

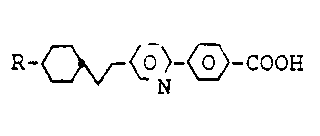

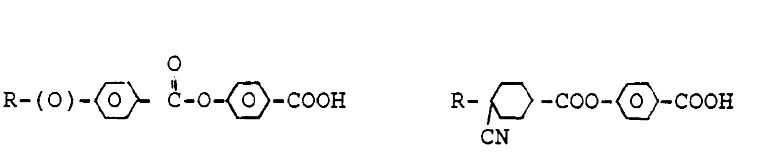

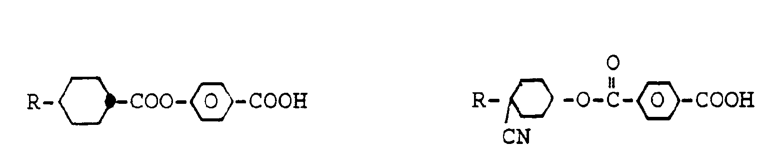

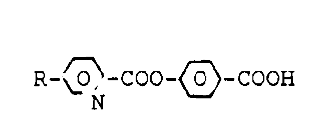

Die erfindungsgemäßen Phasen enthalten mindestens eine, vorzugsweise mindestens zwei Verbindungen der Formel Besonders bevorzugt sind erfindungsgemäße chirale getiltete smektische flüssigkristalline Phasen, deren achirale Basismischung neben Verbindungen der Formel mindestens eine andere Komponente mit negativer oder betragsmäßig kleiner positiver dietektrischer Anisotropie enthält. Die Chiralität beruht vorzugsweise teilweise oder vollständig auf chiralen Verbindungen der Formel I. Diese Phasen enthalten vorzugseise eine oder zwei chirale Verbindungen der Formel Es können jedoch auch achirale Verbindungen der Formel (zum Beispiel in Form eines Racemates) eingesetzt werden, wobei dann die Chiralität der Phase durch andere optisch aktive Verbindungen hervorgerufen wird. Falls chirale Verbindungen der Formel I zum Einsatz kommen, eignen sich neben den reinen optischen Antipoden auch Gemische mit einem Enantiomerenüberschuß. Die oben erwähnten weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp:

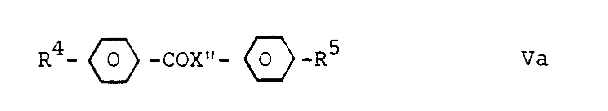

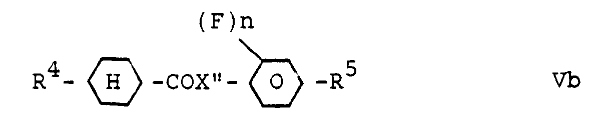

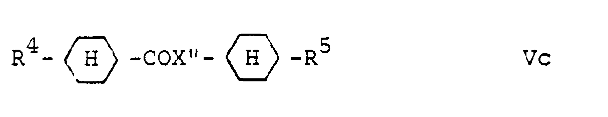

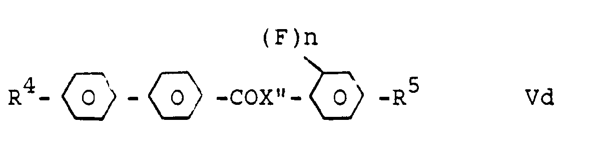

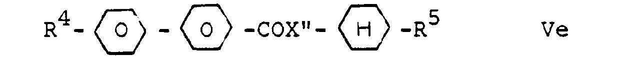

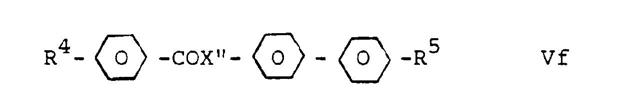

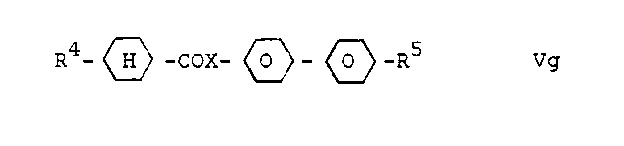

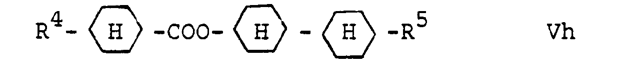

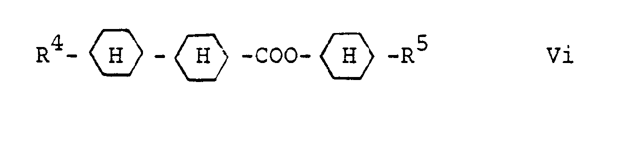

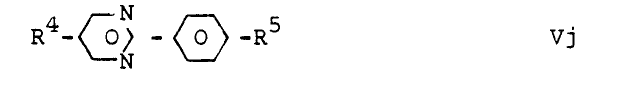

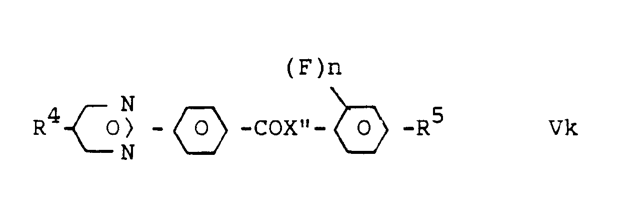

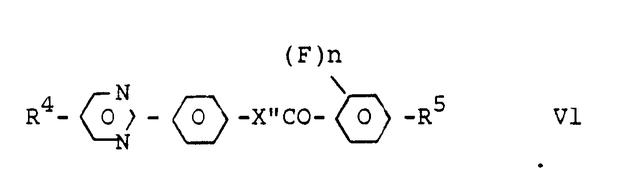

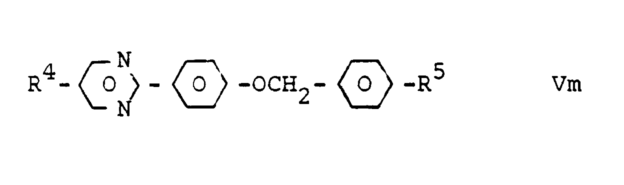

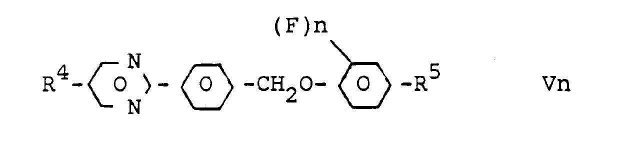

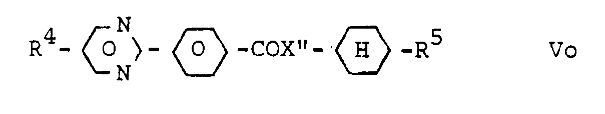

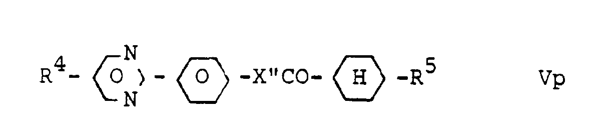

R⁴ und R⁵ sind jeweils vorzugsweise geradkettiges oder verzweigtes Alkyl, Alkoxy, Alkanoyloxy oder Alkoxycarbonyl mit jeweils 3 bis 12 C-Atomen. X" ist O oder S, vorzugsweise O. n ist 0 oder 1.

Besonders bevorzugt sind die Verbindungen der Teilformeln Va, Vb, Vd und Vf, worin R⁴ und R⁵ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. R⁴ und R⁵ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel 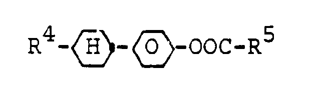 worin R⁴ und R⁵ die für Vc, Vh und Vi angegebene bevorzugte Bedeutung haben.

Als weitere Komponenten mit negativer dielektrischer Anisotropie eignen sich weiterhin Verbindungen enthaltend das Strukturelement M, N oder O.

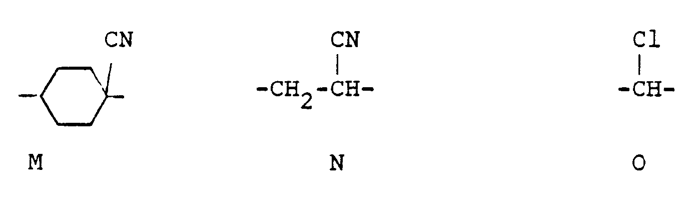

Bevorzugte Verbindungen dieser Art entsprechen den Formeln Vlb und Vlc:

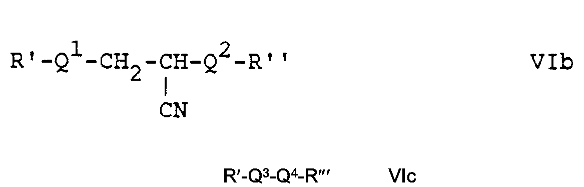

R' und R" bedeuten jeweils vorzugsweise geradkettige Alkyl- oder Alkoxy-Gruppen mit jeweils 2 bis 10 C-Atomen. Q¹ und Q² bedeuten jeweils 1,4-Phenylen, trans-1,4-Cyclohexylen, 4,4'-Biphenylyl, 4-(trans-4-Cyclohexyl)phenyl, trans,trans-4,4'-Bicyclohexyl oder eine der Gruppen Q¹ und Q² auch eine Einfachbindung.

Q³ und Q⁴ bedeuten jeweils 1,4-Phenylen, 4,4'-Biphenylyl oder trans-1,4-Cyclohexylen. Eine der Gruppen Q³ und Q⁴ kann auch 1,4-Phenylen bedeuten, worin mindestens eine CH-Gruppe durch N ersetzt ist. R"' ist ein optisch aktiver Rest mit einem asymmetrischen Kohlenstoffatom der Struktur 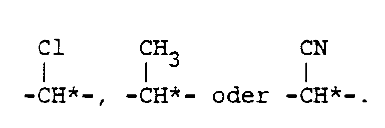 R"' hat vorzugsweise die Formel 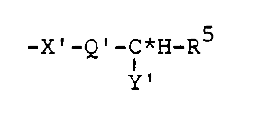 mit den vorgehend genannten bevorzugten Bedeutungen.

Besonders bevorzugte Komponenten mit negativer dielektrischer Anisotropie sind die in der WO 86-00529 beschriebenen Verbindungen mit dem Strukturelement M oder N. Besonders bevorzugt sind diejenigen der Formel Vld 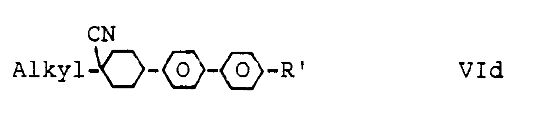 worin Alkyl eine geradkettige oder verzweigte Alkylgruppe mit vorzugsweise 3 bis 10 C-Atomen ist und R' die oben angegebene Bedeutung hat. Ferner bevorzugt sind Verbindungen entsprechend der Formel Vld, worin eine oder beide die Ringe verknüpfenden Einfachbindungen durch eine Gruppe ausgewählt aus -CH₂CH₂-, -O-CO- oder -CO-O-ersetzt sind. Besonders bevorzugte Verbindungen der Formel Vlc sind diejenigen der Formel Vlc': 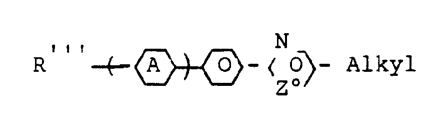 worin A 1,4-Phenylen oder trans-1,4-Cyclohexylen, Z° CH oder N und n 0 oder 1 bedeutet.

Die Verbindungen der Formel eignen sich auch als Komponenten nematischerflüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel 1. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I" charakterisieren,
R'-L-G-E-R" I"

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

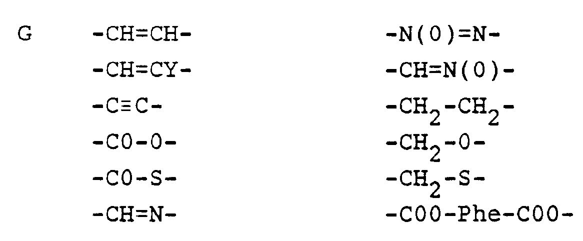

oder eine C-C-Einfachbindung,

Y Halogen, vorzugsweise Chlor, oder-CN, und R' und R" Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, NO₂, CF₃, F, CI oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Es bedeuten ferner:

K: Kristall in-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

### Beispiel 1

Unter Feuchtigkeitsausschluß werden bei -10 ° 25,6 g 2-p-Hydroxyphenyl-5-n-hexylpyrimidin, 19,2 g optisch aktive 2-Ethyl-2-hexylcyanessigsäure und 1,2 g 4-N,N'-Dimethylaminopyridin in 150 ml Methylenchlorid vorgelegt und 22,7 g Dicyclohexylcarbodiimid in 20 ml Methylenchlorid zugetropft. Man rührt unter langsamem Erwärmen auf Raumtemperatur über Nacht, filtriert den ausgefallenen Dicyclohexylharnstoff ab, wäscht die organische Phase mit verdünnter Salzsäure und dann mehrmals mit Wasser. Nach dem Trocknen über Natriumsulfat wird das Lösungsmittel entfernt und der Rückstand chromatographisch und durch Kristallisation gereinigt. Man erhält optisch aktiven 2-Ethyl-2-hexylcyanessigsäure-p-(5-hexylpyrimidin-2-yl)-phenylester.
Analog werden hergestellt:
2-Ethyl-2-hexylcyanessigsäure-p-(5-heptylpyrimidin-2-yl)-phenylester
   2-Ethyl-2-hexylcyanessigsäure-p-(5-pentylpyrimidin-2-yl)-phenylester
   2-Ethyl-2-hexylcyanessigsäure-p-(5-octylpyrimidin-2-yl)-phenylester
   2-Ethyl-2-hexylcyanessigsäure-p-(5-nonylpyrimidin-2-yl)-phenylester
   2-Ethyl-2-hexylcyanessigsäure-p-(5-decylpyrimidin-2-yl)-phenylester
   2-Ethyl-2-hexylcyanessigsäure-p-(5-undecylpyrimidin-2-yl)-phenylester
   2-Ethyl-2-hexylcyanessigsäure-p-(5-dodecylpyrimidin-2-yl)-phenylester
   2-Methyl-2-hexylcyanessigsäure-p-(5-pentylpyrimidin-2-yl)-phenylester
   2-Methyl-2-hexylcyanessigsäure-p-(5-hexylpyrimidin-2-yl)-phenylester
   2-Methyl-2-hexylcyanessigsäure-p-(5-heptylpyrimidin-2-yl)-phenylester
   2-Methyl-2-hexylcyanessigsäure-p-(5-octylpyrimidin-2-yl)-phenylester
   2-Methyl-2-hexylcyanessigsäure-p-(5-nonylpyrimidin-2-yl)-phenylester
   2-Methyl-2-hexylcyanessigsäure-p-(5-decylpyrimidin-2-yl)-phenylester
   2-Methyl-2-hexylcyanessigsäure-p-(5-undecylpyrimidin-2-yl)-phenylester
   2-Methyl-2-hexylcyanessigsäure-p-(5-dodecylpyrimid in-2-yl)-phenylester
2-Methyl-2-pentylcyanessigsäure-p-(5-pentylpyrimidin-2-yl)-phenylester
   2-Methyl-2-pentylcyanessigsäure-p-(5-hexylpyrimidin-2-yl)-phenylester
   2-Methyl-2-pentylcyanessigsäure-p-(5-heptylpyrimidin-2-yl)-phenylester
   2-Methyl-2-pentylcyanessigsäure-p-(5-octylpyrimidin-2-yl)-phenylester
   2-Methyl-2-pentylcyanessigsäure-p-(5-nonylpyrimidin-2-yl)-phenylester
   2-Methyl-2-pentylcyanessigsäure-p-(5-decylpyrimidin-2-yl)-phenylester
   2-Methyl-2-pentylcyanessigsäure-p-(5-undecylpyrimidin-2-yl)-phenylester
   2-Methyl-2-pentylcyanessigsäure-p-(5-dodecylpyrimidin-2-yl)-phenylester
2-Methyl-2-butylcyanessigsäure-p-(5-pentylpyrimidin-2-yl)-phenylester
   2-Methyl-2-butylcyanessigsäure-p-(5-hexylpyrimidin-2-yl)-phenylester
   2-Methyl-2-butylcyanessigsäure-p-(5-heptylpyrimidin-2-yl)-phenylester
   2-Methyl-2-butylcyanessigsäure-p-(5-octylpyrimidin-2-yl)-phenylester
   2-Methyl-2-butylcyanessigsäure-p-(5-nonylpyrimidin-2-yl)-phenylester
   2-Methyl-2-butylcyanessigsäure-p-(5-decylpyrimidin-2-yl)-phenylester
   2-Methyl-2-butylcyanessigsäure-p-(5-undecylpyrimidin-2-yl)-phenylester
   2-Methyl-2-butylcyanessigsäure-p-(5-dodecylpyrimidin-2-yl)-phenylester
2-Methyl-2-propylcyanessigsäure-p-(5-pentylpyrimidin-2-yl)-phenylester
   2-Methyl-2-propylcyanessigsäure-p-(5-hexylpyrimidin-2-yl)-phenylester
   2-Methyl-2-propylcyanessigsäure-p-(5-heptylpyrimidin-2-yl)-phenylester
   2-Methyl-2-propylcyanessigsäure-p-(5-octylpyrimidin-2-yl)-phenylester
   2-Methyl-2-propylcyanessigsäure-p-(5-nonylpyrimidin-2-yl)-phenylester
   2-Methyl-2-propylcyanessigsäure-p-(5-decylpyrimidin-2-yl)-phenylester
   2-Methyl-2-propylcyanessigsäure-p-(5-undecylpyrimidin-2-yl)-phenylester
   2-Methyl-2-propylcyanessigsäure-p-(5-dodecylpyrimidin-2-yl)-phenylester
2-Methyl-2-ethylcyanessigsäure-p-(5-pentylpyrimidin-2-yl)-phenylester
   2-Methyl-2-ethylcyanessigsäure-p-(5-hexylpyrimidin-2-yl)-phenylester
   2-Methyl-2-ethylcyanessigsäure-p-(5-heptylpyrimidin-2-yl)-phenylester
   2-Methyl-2-ethylcyanessigsäure-p-(5-octylpyrimidin-2-yl)-phenylester
   2-Methyl-2-ethylcyanessigsäure-p-(5-nonylpyrimidin-2-yl)-phenylester
   2-Methyl-2-ethylcyanessigsäure-p-(5-decylpyrimidin-2-yl)-phenylester
   2-Methyl-2-ethylcyanessigsäure-p-(5-undecylpyrimidin-2-yl)-phenylester
   2-Methyl-2-ethylcyanessigsäure-p-(5-dodecylpyrimidin-2-yl)-phenylester
2-Methyl-2-isopropylcyanessigsäure-p-(5-pentylpyrimidin-2-yl)-phenylester
   2-Methyl-2-isopropylcyanessigsäure-p-(5-hexylpyrimidin-2-yl)-phenylester
   2-Methyl-2-isopropylcyanessigsäure-p-(5-heptylpyrimidin-2-yl)-phenylester
   2-Methyl-2-isopropylcyanessigsäure-p-(5-octylpyrimidin-2-yl)-phenylester
   2-Methyl-2-isopropylcyanessigsäure-p-(5-nonylpyrimidin-2-yl)-phenylester
   2-Methyl-2-isopropylcya ness igsäure-p-(5-decyl pyrimid i n-2-yl)-p henylester
   2-Methyl-2-isopropylcyanessigsäure-p-(5-undecylpyrimidin-2-yl)-phenylester
   2-Methyl-2-isopropylcyanessigsäure-p-(5-dodecylpyrimidin-2-yl)-phenylester
2-Methyl-2-isobutylcyanessigsäure-p-(5-pentylpyrimidin-2-yl)-phenylester
   2-Methyl-2-isobutylcyanessigsäure-p-(5-hexylpyrimidin-2-yl)-phenylester
   2-Methyl-2-isobutylcyanessigsäure-p-(5-heptylpyrimid in-2-yl)-phenylester
   2-Methyl-2-isobutylcyanessigsäure-p-(5-octylpyrimidin-2-yl)-phenylester
   2-Methyl-2-isobutylcyanessigsäure-p-(5-nonylpyrimidin-2-yl)-phenylester
   2-Methyl-2-isobutylcyanessigsäure-p-(5-decylpyrimidin-2-yl)-phenylester
   2-Methyl-2-isobutylcyanessigsäure-p-(5-undecylpyrimidin-2-yl)-phenylester
   2-Methyl-2-isobutylcyanessigsäure-p-(5-dodecylpyrimidin-2-yl)-phenylester
2-Methyl-2-(2-methylbutyl)-cyanessigsäure-p-(5-pentylpyrimidin -2-yl)-phenylester
   2-Methyl-2-(2-methylbutyl)-cyanessigsäure-p-(5-hexylpyrimidin -2-yl)-phenylester
   2-Methyl-2-(2-methylbutyl)-cyanessigsäure-p-(5-heptylpyrimidin -2-yl)-phenylester
   2-Methyl-2-(2-methylbutyl)-cyanessigsäure-p-(5-octylpyrimidin -2-yl)-phenylester
   2-Methyl-2-(2-methylbutyl)-cyanessigsäure-p-(5-nonylpyrimidin -2-yl)-phenylester
   2-Methyl-2-(2-methylbutyl)-cyanessigsäure-p-(5-decylpyrimidin -2-yl)-phenylester
   2-Methyl-2-(2-methylbutyl)-cyanessigsäure-p-(5-undecylpyrimidin -2-yl)-phenylester
   2-Methyl-2-(2-methylbutyl)-cyanessigsäure-p-(5-dodecylpyrimidin -2-yl)-phenylester
2-Methyl-2-ethylcyanessigsäure-p-(5-pentylpyridin-2-yl)-phenylester
   2-Methyl-2-ethylcyanessigsäure-p-(5-hexylpyridin-2-yl)-phenylester
   2-Methyl-2-ethylcyanessigsäure-p-(5-heptylpyridin-2-yl)-phenylester
   2-Methyl-2-ethylcyanessigsäure-p-(5-octylpyridin-2-yl)-phenylester
   2-Methyl-2-ethylcyanessigsäure-p-(5-nonylpyridin-2-yl)-phenylester
   2-Methyl-2-ethylcyanessigsäure-p-(5-decylpyridin-2-yl)-phenylester
   2-Methyl-2-ethylcyanessigsäure-p-(5-undecylpyridin-2-yl)-phenylester
   2-Methyl-2-ethylcyanessigsäure-p-(5-dodecylpyridin-2-yl)-phenylester
2-Methyl-2-propylcyanessigsäure-p-(5-pentylpyridin-2-yl)-phenylester
   2-Methyl-2-propylcyanessigsäure-p-(5-hexylpyridin-2-yl)-phenylester
   2-Methyl-2-propylcyanessigsäure-p-(5-heptylpyridin-2-yl)-phenylester
   2-Methyl-2-propylcyanessigsäure-p-(5-octylpyridin-2-yl)-phenylester
   2-Methyl-2-propylcyanessigsäure-p-(5-nonylpyridin-2-yl)-phenylester
   2-Methyl-2-propylcyanessigsäure-p-(5-decylpyridin-2-yl)-phenylester
   2-Methyl-2-propylcyanessigsäure-p-(5-undecylpyridin-2-yl)-phenylester
   2-Methyl-2-propylcyanessigsäure-p-(5-dodecylpyridin-2-yl)-phenylester
2-Methyl-2-isopropylcyanessigsäure-p-(5-pentylpyridin-2-yl)-phenylester
   2-Methyl-2-isopropylcyanessigsäure-p-(5-hexylpyridin-2-yl)-phenylester
   2-Methyl-2-isopropylcyanessigsäure-p-(5-heptylpyridin-2-yl)-phenylester
   2-Methyl-2-isopropylcyanessigsäure-p-(5-octylpyridin-2-yl)-phenylester
   2-Methyl-2-isopropylcyanessigsäure-p-(5-nonylpyridin-2-yl)-phenylester
   2-Methyl-2-isopropylcyanessigsäure-p-(5-decylpyridin-2-yl)-phenylester
   2-Methyl-2-isopropylcyanessigsäure-p-(5-undecylpyridin-2-yl)-phenylester
   2-Methyl-2-isopropylcyanessigsäure-p-(5-dodecylpyridin-2-yl)-phenylester
2-Methyl-2-butylcyanessigsäure-p-(5-pentylpyridin-2-yl)-phenylester
   2-Methyl-2-butylcyanessigsäure-p-(5-hexylpyridin-2-yl)-phenylester
   2-Methyl-2-butylcyanessigsäure-p-(5-heptylpyridin-2-yl)-phenylester
   2-Methyl-2-butylcyanessigsäure-p-(5-octylpyridin-2-yl)-phenylester
   2-Methyl-2-butylcyanessigsäure-p-(5-nonylpyridin-2-yl)-phenylester
   2-Methyl-2-butylcyanessigsäure-p-(5-decylpyridin-2-yl)-phenylester
   2-Methyl-2-butylcyanessigsäure-p-(5-undecylpyridin-2-yl)-phenylester
   2-Methyl-2-butylcyanessigsäure-p-(5-dodecylpyridin-2-yl)-phenylester
2-Methyl-2-isobutylcyanessigsäure-p-(5-pentylpyridin-2-yl)-phenylester
   2-Methyl-2-isobutylcyanessigsäure-p-(5-hexylpyridin-2-yl)-phenylester
   2-Methyl-2-isobutylcyanessigsäure-p-(5-heptylpyridin-2-yl)-phenylester
   2-Methyl-2-isobutylcyanessigsäure-p-(5-octylpyridin-2-yl)-phenylester
   2-Methyl-2-isobutylcyanessigsäure-p-(5-nonylpyridin-2-yl)-phenylester
   2-Methyl-2-isobutylcyanessigsäure-p-(5-decylpyridin-2-yl)-phenylester
   2-Methyl-2-isobutylcyanessigsäure-p-(5-undecylpyridin-2-yl)-phenylester
   2-Methyl-2-isobutylcyanessigsäure-p-(5-dodecylpyridin-2-yl)-phenylester
2-Methyl-2-(2-methylbutyl)-cyanessigsäure-p-(5-pentylpyridin-2-yl)-phenylester
   2-Methyl-2-(2-methylbutyl)-cyanessigsäure-p-(5-hexylpyridin-2-yl)-phenylester
   2-Methyl-2-(2-methylbutyl)-cyanessigsäure-p-(5-heptylpyridin-2-yl)-phenylester
   2-Methyl-2-(2-methylbutyl)-cyanessigsäure-p-(5-octylpyridin-2-yl)-phenylester
   2-Methyl-2-(2-methylbutyl)-cyanessigsäure-p-(5-nonylpyridin-2-yl)-phenylester
   2-Methyl-2-(2-methylbutyl)-cyanessigsäure-p-(5-decylpyridin-2-yl)-phenylester
   2-Methyl-2-(2-methylbutyl)-cyanessigsäure-p-(5-undecylpyridin-2-yl)-phenylester
   2-Methyl-2-(2-methylbutyl)-cyanessigsäure-p-(5-dodecylpyridin-2-yl)-phenylester
2-Methyl-2-pentylcyanessigsäure-p-(5-pentylpyridin-2-yl)-phenylester
   2-Methyl-2-pentylcyanessigsäure-p-(5-hexylpyridin-2-yl)-phenylester
   2-Methyl-2-pentylcyanessigsäure-p-(5-heptylpyridin-2-yl)-phenylester
   2-Methyl-2-pentylcyanessigsäure-p-(5-octylpyridin-2-yl)-phenylester
   2-Methyl-2-pentylcyanessigsäure-p-(5-nonylpyridin-2-yl)-phenylester
   2-Methyl-2-pentylcyanessigsäure-p-(5-decylpyridin-2-yl)-phenylester
   2-Methyl-2-pentylcyanessigsäure-p-(5-undecylpyridin-2-yl)-phenylester
   2-Methyl-2-pentylcyanessigsäure-p-(5-dodecylpyridin-2-yl)-phenylester
2-Methyl-2-hexylcyanessigsäure-p-(5-pentylpyridin-2-yl)-phenylester
   2-Methyl-2-hexylcyanessigsäure-p-(5-hexylpyridin-2-yl)-phenylester
   2-Methyl-2-hexylcyanessigsäure-p-(5-heptylpyridin-2-yl)-phenylester
   2-Methyl-2-hexylcyanessigsäure-p-(5-octylpyridin-2-yl)-phenylester
   2-Methyl-2-hexylcyanessigsäure-p-(5-nonylpyridin-2-yl)-phenylester
   2-Methyl-2-hexylcyanessigsäure-p-(5-decylpyridin-2-yl)-phenylester
   2-Methyl-2-hexylcyanessigsäure-p-(5-undecylpyridin-2-yl)-phenylester
   2-Methyl-2-hexylcyanessigsäure-p-(5-dodecylpyridin-2-yl)-phenylester
2-Methyl-2-ethylcyanessigsäure-4'-butyloxybiphenyl-4-ylester
   2-Methyl-2-ethylcyanessigsäure-4'-pentyloxybiphenyl-4-ylester
   2-Methyl-2-ethylcyanessigsäure-4'-hexyloxybiphenyl-4-ylester
   2-Methyl-2-ethylcyanessigsäure-4'-heptyloxybiphenyl-4-ylester
   2-Methyl-2-ethylcyanessigsäure-4'-octyloxybiphenyl-4-ylester
   2-Methyl-2-ethylcyanessigsäure-4'-nonyloxybiphenyl-4-ylester
   2-Methyl-2-ethylcyanessigsäure-4'-decyloxybiphenyl-4-ylester
   2-Methyl-2-ethylcyanessigsäure-4'-undecyloxybiphenyl-4-ylester
   2-Methyl-2-ethylcyanessigsäure-4'-dodecyloxybiphenyl-4-ylester
2-Methyl-2-ethylcyanessigsäure-4'-pentylbiphenyl-4-ylester
   2-Methyl-2-ethylcyanessigsäure-4'-hexylbiphenyl-4-ylester
   2-Methyl-2-ethylcyanessigsäure-4'-heptylbiphenyl-4-ylester
   2-Methyl-2-ethylcyanessigsäure-4'-octylbiphenyl-4-ylester
   2-Methyl-2-ethylcyanessigsäure-4'-nonylbiphenyl-4-ylester
   2-Methyl-2-ethylcyanessigsäure-4'-decylbiphenyl-4-ylester
   2-Methyl-2-ethylcyanessigsäure-4'-undecylbiphenyl-4-ylester
   2-Methyl-2-ethylcyanessigsäure-4'-dodecylbiphenyl-4-ylester
   2-Methyl-2-ethylcyanessigsäure-4'-(5-pentylpyrimidin-2-yl)-biphenyl-4-ylester
2-Methyl-2-ethylcyanessigsäure-4'-(5-hexylpyrimidin-2-yl)-biphenyl-4-ylester
   2-Methyl-2-ethylcyanessigsäure-4'-(5-heptylpyrimidin-2-yl)-biphenyl-4-ylester
   2-Methyl-2-ethylcyanessigsäure-4'-(5-octylpyrimidin-2-yl)-biphenyl-4-ylester
   2-Methyl-2-ethylcyanessigsäure-4'-(5-nonylpyrimidin-2-yl)-biphenyl-4-ylester
   2-Methyl-2-ethylcyanessigsäure-4'-(5-decylpyrimidin-2-yl)-biphenyl-4-ylester
   2-Methyl-2-ethylcyanessigsäure-4'-(5-undecylpyrimidin-2-yl)-biphenyl-4-ylester
   2-Methyl-2-ethylcyanessigsäure-4'-(5-dodecylpyrimidin-2-yl)-biphenyl-4-ylester
2-Methyl-2-ethylcyanessigsäure-4'-(5-pentylpyridin-2-yl)-biphenyl-4-ylester
   2-Methyl-2-ethylcyanessigsäure-4'-(5-hexylpyridin-2-yl)-biphenyl-4-ylester
   2-Methyl-2-ethylcyanessigsäure-4'-(5-heptylpyridin-2-yl)-biphenyl-4-ylester
   2-Methyl-2-ethylcyanessigsäure-4'-(5-octylpyridin-2-yl)-biphenyl-4-ylester
   2-Methyl-2-ethylcyanessigsäure-4'-(5-nonylpyridin-2-yl)-biphenyl-4-ylester
   2-Methyl-2-ethylcyanessigsäure-4'-(5-decylpyridin-2-yl)-biphenyl-4-ylester
   2-Methyl-2-ethylcyanessigsäure-4'-(5-undecylpyridin-2-yl)-biphenyl-4-ylester
   2-Methyl-2-ethylcyanessigsäure-4'-(5-dodecylpyridin-2-yl)-biphenyl-4-ylester
2-Methyl-2-ethylcyanessigsäure-4'-(trans-4-pentylcyclohexyl)-biphenyl-4-ylester
   2-Methyl-2-ethylcyanessigsäure-4'-(trans-4-hexylcyclohexyl)-biphenyl-4-ylester
   2-Methyl-2-ethylcyanessigsäure-4'-(trans-4-heptylcyclohexyl)-biphenyl-4-ylester
   2-Methyl-2-ethylcyanessigsäure-4'-(trans-4-octylcyclohexyl)-biphenyl-4-ylester
   2-Methyl-2-ethylcyanessigsäure-4'-(trans-4-nonylcyclohexyl)-biphenyl-4-ylester
   2-Methyl-2-ethylcyanessigsäure-4'-(trans-4-decylcyclohexyl)-biphenyl-4-ylester
   2-Methyl-2-ethylcyanessigsäure-4'-(trans-4-undecylcyclohexyl)-biphenyl-4-ylester
   2-Methyl-2-ethylcyanessigsäure-4'-(trans-4-dodecylcyclohexyl)-biphenyl-4-ylester
2-Methyl-2-propylcyanessigsäure-p-[5-(trans-4-pentylcyclohexyl)-pyrimidin-2-yl]-phenylester
   2-Methyl-2-propylcyanessigsäure-p-[5-(trans-4-hexylcyclohexyl)-pyrimidin-2-yl]-phenylester
   2-Methyl-2-propylcyanessigsäure-p-[5-(trans-4-heptylcyclohexyl)-pyrimidin-2-yl]-phenylester
   2-Methyl-2-propylcyanessigsäure-p-[5-(trans-4-octylcyclohexyl)-pyrimidin-2-yl]-phenylester
   2-Methyl-2-propylcyanessigsäure-p-[5-(trans-4-nonylcyclohexyl)-pyrimidin-2-yl]-phenylester
   2-Methyl-2-propylcyanessigsäure-p-[5-(trans-4-decylcyclohexyl)-pyrimidin-2-yl]-phenylester
   2-Methyl-2-propylcyanessigsäure-p-[5-(trans-4-undecylcyclohexyl)-pyrimidin-2-yl]-phenylester
   2-Methyl-2-propylcyanessigsäure-p-[5-(trans-4-dodecylcyclohexyl)-pyrimidin-2-yl]-phenylester
2-Methyl-2-ethylcyanessigsäure-p-[5-(trans-4-pentylcyclohexyl)-pyridin-2-yl]-phenylester
   2-Methyl-2-ethylcyanessigsäure-p-[5-(trans-4-hexylcyclohexyl)-pyridin-2-yl]-phenylester
   2-Methyl-2-ethylcyanessigsäure-p-[5-(trans-4-heptylcyclohexyl)-pyridin-2-yl]-phenylester
   2-Methyl-2-ethylcyanessigsäure-p-[5-(trans-4-octylcyclohexyl)-pyridin-2-yl]-phenylester
   2-Methyl-2-ethylcyanessigsäure-p-[5-(trans-4-nonylcyclohexyl)-pyridin-2-yl]-phenylester
   2-Methyl-2-ethylcyanessigsäure-p-[5-(trans-4-decylcyclohexyl)-pyridin-2-yl]-phenylester
   2-Methyl-2-ethylcyanessigsäure-p-[5-(trans-4-undecylcyclohexyl)-pyridin-2-yl]-phenylester
   2-Methyl-2-ethylcyanessigsäure-p-[5-(trans-4-dodecylcyclohexyl)-pyridin-2-yl]-phenylester
2-Methyl-2-propylcyanessigsäure-p-[5-{2-(trans-4-pentylcyclohexyl)-ethyl}-pyridin-2-yl]-phenylester
   2-Methyl-2-propylcyanessigsäure-p-[5-{2-(trans-4-hexylcyclohexyl)-ethyl}-pyridin-2-yl]-phenylester
   2-Methyl-2-propylcyanessigsäure-p-[5-{2-(trans-4-heptylcyclohexyl)-ethyl}-pyridin-2-yl]-phenylester
   2-Methyl-2-propylcyanessigsäure-p-[5-{2-(trans-4-octylcyclohexyl)-ethyl}-pyridin-2-yl]-phenylester
   2-Methyl-2-propylcyanessigsäure-p-[5-{2-(trans-4-nonylcyclohexyl)-ethyl}-pyridin-2-yl]-phenylester
   2-Methyl-2-propylcyanessigsäure-p-[5-{2-(trans-4-decylcyclohexyl)-ethyl}-pyridin-2-yl]-phenylester
   2-Methyl-2-propylcyanessigsäure-p-[5-{2-(trans-4-undecylcyclohexyl)-ethyl}-pyridin-2-yl]-phenylester
   2-Methyl-2-propylcyanessigsäure-p-[5-{2-(trans-4-dodecylcyclohexyl)-ethyl}-pyridin-2-yl]-phenylester
   2-Methyl-2-propylcyanessigsäure-p-(p-pentyl-benzoyloxy)-phenylester
   2-Methyl-2-propylcyanessigsäure-p-(p-hexyl-benzoyloxy)-phenylester
   2-Methyl-2-propylcyanessigsäure-p-(p-heptyl-benzoyloxy)-phenylester
   2-Methyl-2-propylcyanessigsäure-p-(p-octyl-benzoyloxy)-phenylester
   2-Methyl-2-propylcyanessigsäure-p-(p-nonyl-benzoyloxy)-phenylester
   2-Methyl-2-propylcyanessigsäure-p-(p-decyl-benzoyloxy)-phenylester
   2-Methyl-2-propylcyanessigsäure-p-(p-undecyl-benzoyloxy)-phenylester
   2-Methyl-2-propylcyanessigsäure-p-(p-dodecyl-benzoyloxy)-phenylester
2-Methyl-2-propylcyanessigsäure-p-(p-pentyloxy-benzoyloxy)-phenylester
   2-Methyl-2-propylcyanessigsäure-p-(p-hexyloxy-benzoyloxy)-phenylester
   2-Methyl-2-propylcyanessigsäure-p-(p-heptyloxy-benzoyloxy)-phenylester
   2-Methyl-2-propylcyanessigsäure-p-(p-octyloxy-benzoyloxy)-phenylester
   2-Methyl-2-propylcyanessigsäure-p-(p-nonyloxy-benzoyloxy)-phenylester
   2-Methyl-2-propylcyanessigsäure-p-(p-decyloxy-benzoyloxy)-phenylester
   2-Methyl-2-propylcyanessigsäure-p-(p-undecyloxy-benzoyloxy)-phenylester
   2-Methyl-2-propylcyanessigsäure-p-(p-dodecyloxy-benzoyloxy)-phenylester
2-Methyl-2-ethylcyanessigsäure-p-[p-(p-pentylphenyl)-benzoyloxy]-phenylester
   2-Methyl-2-ethylcyanessigsäure-p-[p-(p-hexylphenyl)-benzoyloxy]-phenylester
   2-Methyl-2-ethylcyanessigsäure-p-[p-(p-heptylphenyl)-benzoyloxy]-phenylester
   2-Methyl-2-ethylcyanessigsäure-p-[p-(p-octylphenyl)-benzoyloxy]-phenylester
   2-Methyl-2-ethylcyanessigsäure-p-[p-(p-nonylphenyl)-benzoyloxy]-phenylester
   2-Methyl-2-ethylcyanessigsäure-p-[p-(p-decylphenyl)-benzoyloxy]-phenylester
   2-Methyl-2-ethylcyanessigsäure-p-[p-(p-undecylphenyl)-benzoyloxy]-phenylester
   2-Methyl-2-ethylcyanessigsäure-p-[p-(p-dodecylphenyl)-benzoyloxy]-phenylester
2-Methyl-2-ethylcyanessigsäure-p-[p-(p-pentyloxyphenyl)benzoyloxy]-phenylester
   2-Methyl-2-ethylcyanessigsäure-p-[p-(p-hexyloxyphenyl)benzoyloxy]-phenylester
   2-Methyl-2-ethylcyanessigsäure-p-[p-(p-heptyloxyphenyl)benzoyloxy]-phenylester
   2-Methyl-2-ethylcyanessigsäure-p-[p-(p-octyloxyphenyl)benzoyloxy]-phenylester
   2-Methyl-2-ethylcyanessigsäure-p-[p-(p-nonyloxyphenyl)benzoyloxy]-phenylester
   2-Methyl-2-ethylcyanessigsäure-p-[p-(p-decyloxyphenyl)benzoyloxy]-phenylester
   2-Methyl-2-ethylcyanessigsäure-p-[p-(p-undecyloxyphenyl)benzoyloxy]-phenylester
   2-Methyl-2-ethylcyanessigsäure-p-[p-(p-dodecyloxyphenyl)benzoyloxy]-phenylester
2-Methyl-2-propylcyanessigsäure-4'-(p-pentylbenzoyloxy)biphenyl-4-ylester
   2-Methyl-2-propylcyanessigsäure-4'-(p-hexylbenzoyloxy)biphenyl-4-ylester
   2-Methyl-2-propylcyanessigsäure-4'-(p-heptylbenzoyloxy)biphenyl-4-ylester
   2-Methyl-2-propylcyanessigsäure-4'-(p-octylbenzoyloxy)biphenyl-4-ylester
   2-Methyl-2-propylcyanessigsäure-4'-(p-nonylbenzoyloxy)biphenyl-4-ylester
   2-Methyl-2-propylcyanessigsäure-4'-(p-decylbenzoyloxy)biphenyl-4-ylester
   2-Methyl-2-propylcyanessigsäure-4'-(p-undecylbenzoyloxy)biphenyl-4-ylester
   2-Methyl-2-propylcyanessigsäure-4'-(p-dodecylbenzoyloxy)biphenyl-4-ylester
2-Methyl-2-propylcyanessigsäure-4'-(p-pentyloxybenzoyloxy)- biphenyl-4-ylester
   2-Methyl-2-propylcyanessigsäure-4'-(p-hexyloxybenzoyloxy)-biphenyl-4-ylester
   2-Methyl-2-propylcyanessigsäure-4'-(p-heptyloxybenzoyloxy)-biphenyl-4-ylester
   2-Methyl-2-propylcyanessigsäure-4'-(p-octyloxybenzoyloxy)-biphenyl-4-ylester
   2-Methyl-2-propylcyanessigsäure-4'-(p-nonyloxybenzoyloxy)-biphenyl-4-ylester
   2-Methyl-2-propylcyanessigsäure-4'-(p-decyloxybenzoyloxy)-biphenyl-4-ylester
   2-Methyl-2-propylcyanessigsäure-4'-(p-undecyloxybenzoyloxy)-biphenyl-4-ylester
   2-Methyl-2-propylcyanessigsäure-4'-(p-dodecyloxybenzoyloxy)-biphenyl-4-ylester
2-Methyl-2-ethylcyanessigsäure-p-(trans-4-pentylcyclohexylcarbonyloxy)-phenylester
   2-Methyl-2-ethylcyanessigsäure-p-(trans-4-hexylcyclohexylcarbonyloxy)-phenylester
   2-Methyl-2-ethylcyanessigsäure-p-(trans-4-heptylcyclohexylcarbonyloxy)-phenylester
   2-Methyl-2-ethylcyanessigsäure-p-(trans-4-octylcyclohexylcarbonyloxy)-phenylester
   2-Methyl-2-ethylcyanessigsäure-p-(trans-4-nonylcyclohexylcarbonyloxy)-phenylester
   2-Methyl-2-ethylcyanessigsäure-p-(trans-4-decylcyclohexylcarbonyloxy)-phenylester
   2-Methyl-2-ethylcyanessigsäure-p-(trans-4-undecylcyclohexylcarbonyloxy)-phenylester
   2-Methyl-2-ethylcyanessigsäure-p-(trans-4-dodecylcyclohexylcarbonyloxy)-phenylester
2-Methyl-2-ethylcyanessigsäure-p-(5-pentylpyridin-2-yl-carbonyloxy)-phenylester
   2-Methyl-2-ethylcyanessigsäure-p-(5-hexylpyridin-2-yl-carbonyloxy)-phenylester
   2-Methyl-2-ethylcyanessigsäure-p-(5-heptylpyridin-2-yl-carbonyloxy)-phenylester
   2-Methyl-2-ethylcyanessigsäure-p-(5-octylpyridin-2-yl-carbonyloxy)-phenylester
   2-Methyl-2-ethylcyanessigsäure-p-(5-nonylpyridin-2-yl-carbonyloxy)-phenylester
   2-Methyl-2-ethylcyanessigsäure-p-(5-decylpyridin-2-yl-carbonyloxy)-phenylester
   2-Methyl-2-ethylcyanessigsäure-p-(5-undecylpyridin-2-yl-carbonyloxy)-phenylester
   2-Methyl-2-ethylcyanessigsäure-p-(5-dodecylpyridin-2-yl-carbonyloxy)-phenylester
2-Methyl-2-ethylcyanessigsäure-p-(trans-4-pentylcyclohexyl)-phenylester
   2-Methyl-2-ethylcyanessigsäure-p-(trans-4-hexylcyclohexyl)-phenylester
   2-Methyl-2-ethylcyanessigsäure-p-(trans-4-heptylcyclohexyl)-phenylester
   2-Methyl-2-ethylcyanessigsäure-p-(trans-4-octylcyclohexyl)-phenylester
   2-Methyl-2-ethylcyanessigsäure-p-(trans-4-nonylcyclohexyl)-phenylester
   2-Methy1-2-ethylcyanessigsäure-p-(trans-4-decylcyclohexyl)-phenylester
   2-Methyl-2-ethylcyanessigsäure-p-(trans-4-u ndecylcyclohexyl)-phenylester
   2-Methyl-2-ethylcyanessigsäure-p-(trans-4-dodecylcyclohexyl)-phenylester
2-Methyl-2-ethylcyanessigsäure-trans-4-(p-pentylphenyl)-cyclohexylester
   2-Methyl-2-ethylcyanessigsäure-trans-4-(p-hexyl phenyl)-cyclohexylester
   2-Methyl-2-ethylcyanessigsäure-trans-4-(p-heptylphenyl)-cyclohexylester
   2-Methyl-2-ethylcyanessigsäure-trans-4-(p-octylphenyl)-cyclohexylester
   2-Methyl-2-ethylcyanessigsäure-trans-4-(p-nonyl phenyl)-cyclohexylester
   2-Methyl-2-ethylcyanessigsäure-trans-4-(p-decyl phenyl)-cyclohexylester
   2-Methyl-2-ethylcyanessigsäure-trans-4-(p-dodecyl phenyl)-cyclohexylester
   2-Methyl-2-ethylcyanessigsäure-trans-4-(p-undecylphenyl)-cyclohexylester
2-Methyl-2-ethylcyanessigsäure-trans-4-(p-pentyloxyphenyl)-cyclohexylester
   2-Methyl-2-ethylcyanessigsäure-trans-4-(p-hexyloxyphenyl)-cyclohexylester
   2-Methyl-2-ethylcyanessigsäure-trans-4-(p-heptyloxyphenyl)-cyclohexylester
   2-Methyl-2-ethylcyanessigsäure-trans-4-(p-octyloxyphenyl)-cyclohexylester
   2-Methyl-2-ethylcyanessigsäure-trans-4-(p-nonyloxyphenyl)-cyclohexylester
   2-Methyl-2-ethylcyanessigsäure-trans-4-(p-decyloxyphenyl)-cyclohexylester
   2-Methyl-2-ethylcyanessigsäure-trans-4-(p-dodecyloxyphenyl)-cyclohexylester
   2-Methyl-2-ethylcyanessigsäure-trans-4-(p-undecyloxyphenyl)-cyclohexylester
2-Methyl-2-ethylcyanessigsäure-trans-4-(4'-pentylbipheny1-4-yl)-cyclohexylester
   2-Methyl-2-ethylcyanessigsäure-trans-4-(4'-hexylbiphenyl-4-yl)-cyclohexylester
   2-Methyl-2-ethylcyanessigsäure-trans-4-(4'-heptylbiphenyl-4-yl)-cyclohexylester
   2-Methyl-2-ethylcyanessigsäure-trans-4-(4'-octylbiphenyl-4-yl)-cyclohexylester
   2-Methyl-2-ethylcyanessigsäure-trans-4-(4'-nonylbiphenyl-4-yl)-cyclohexylester
   2-Methyl-2-ethylcyanessigsäure-trans-4-(4'-decylbiphenyl-4-yl)-cyclohexylester
   2-Methyl-2-ethylcyanessigsäure-trans-4-(4'-dodecylbiphenyl-4-yl)-cyclohexylester
   2-Methyl-2-ethylcyanessigsäure-trans-4-(4'-undecylbiphenyl-4-yl)-cyclohexylester
2-Methyl-2-ethylcyanessigsäure-trans-4-(4'-pentyloxybiphenyl-4-yl)-cyclohexylester
   2-Methyl-2-ethylcyanessigsäure-trans-4-(4'-hexyloxybiphenyl-4-yl)-cyclohexylester
   2-Methyl-2-ethylcyanessigsäure-trans-4-(4'-heptyloxybiphenyl-4-yl)-cyclohexylester
   2-Methyl-2-ethylcyanessigsäure-trans-4-(4'-octyloxybiphenyl-4-yl)-cyclohexylester
   2-Methyl-2-ethylcyanessigsäure-trans-4-(4'-nonyloxybipheny1-4-yl)-cyclohexylester
   2-Methyl-2-ethylcyanessigsäure-trans-4-(4'-decyloxybiphenyl-4-yl)-cyclohexylester
   2-Methyl-2-ethylcyanessigsäure-trans-4-(4'-dodecyloxybiphenyl-4-yl)-cyclohexylester
   2-Methyl-2-ethylcyanessigsäure-trans-4-(4'-undecyloxybiphenyl-4-yl)-cyclohexylester
2-Methyl-2-propylcyanessigsäure-p-(4-cyan-4-pe ntylcyclohexyl)-phenylester
   2-Methyl-2-propylcyanessigsäure-p-(4-cyan-4-hexylcyclohexyl)-phenylester
   2-Methyl-2-propylcyanessigsäure-p-(4-cyan-4-heptylcyclohexyl)-phenylester
   2-Methyl-2-propylcyanessigsäure-p-(4-cyan-4-octylcyclohexyl)-phenylester
   2-Methyl-2-propylcyanessigsäure-p-(4-cyan-4-nonylcyclohexyl)-phenylester
   2-Methyl-2-propylcyanessigsäure-p-(4-cyan-4-decylcyclohexyl)-phenylester
   2-Methyl-2-propylcyanessigsäure-p-(4-cyan-4-u ndecylcyclohexyl)-phenylester
   2-Methyl-2-propylcyanessigsäure-p-(4-cyan-4-dodecylcyclohexyl)-phenylester
2-Methyl-2-propylcyanessigsäure-4'-(4-cyan-4-pentylcyclohexyl)-biphenyl-4-ylester
   2-Methyl-2-propylcyanessigsäure-4'-(4-cyan-4-hexylcyclohexyl)-biphenyl-4-ylester
   2-Methyl-2-propylcyanessigsäure-4'-(4-cyan-4-heptylcyclohexyl)-biphenyl-4-ylester
   2-Methyl-2-propylcyanessigsäure-4'-(4-cyan-4-octylcyclohexyl)-biphenyl-4-ylester
   2-Methyl-2-propylcyanessigsäure-4'-(4-cyan-4-nonylcyclohexyl)-biphenyl-4-ylester
   2-Methyl-2-propylcyanessigsäure-4'-(4-cyan-4-decylcyclohexyl)-biphenyl-4-ylester
   2-Methyl-2-propylcyanessigsäure-4'-(4-cyan-4-undecylcyclohexyl)-biphenyl-4-ylester
   2-Methyl-2-propylcyanessigsäure-4'-(4-cyan-4-dodecylcyclohexyl)-biphenyl-4-ylester
   2-Methyl-2-ethylcyanessigsäure-p-(4-cyan-4-pentylcyclohexylcarbonyloxy)-phenylester
   2-Methyl-2-ethylcyanessigsäure-p-(4-cyan-4-hexylcyclohexylcarbonyloxy)-phenylester
   2-Methyl-2-ethylcyanessigsäure-p-(4-cyan-4-heptylcyclohexylcarbonyloxy)-phenylester
   2-Methyl-2-ethylcyanessigsäure-p-(4-cyan-4-octylcyclohexylcarbonyloxy)-phenylester
   2-Methyl-2-ethylcyanessigsäure-p-(4-cyan-4-nonylcyclohexylcarbonyloxy)-phenylester
   2-Methyl-2-ethylcyanessigsäure-p-(4-cyan-4-decylcyclohexylcarbonyloxy)-phenylester
   2-Methyl-2-ethylcyanessigsäure-p-(4-cyan-4-undecylcyclohexylcarbonyloxy)-phenylester
   2-Methyl-2-ethylcyanessigsäure-p-(4-cyan-4-dodecylcyclohexylcarbonyloxy)-phenylester
2-Methyl-2-ethylcyanessigsäure-p-(4-cyan-4-pentylcyclohexyloxycarbonyl)-phenylester
   2-Methyl-2-ethylcyanessigsäure-p-(4-cyan-4-hexylcyclohexyloxycarbonyl)-phenylester
   2-Methyl-2-ethylcyanessigsäure-p-(4-cyan-4-heptylcyclohexyloxycarbonyl)-phenylester
   2-Methyl-2-ethylcyanessigsäure-p-(4-cyan-4-octylcyclohexyloxycarbonyl)-phenylester
   2-Methyl-2-ethylcyanessigsäure-p-(4-cyan-4-nonylcyclohexyloxycarbonyl)-phenylester
   2-Methyl-2-ethylcyanessigsäure-p-(4-cyan-4-decylcyclohexyloxycarbonyl)-phenylester
   2-Methyl-2-ethylcyanessigsäure-p-(4-cyan-4-undecylcyclohexyloxycarbonyl)-phenylester
   2-Methyl-2-ethylcyanessigsäure-p-(4-cyan-4-dodecylcyclohexyloxycarbonyl)-phenylester

### Beispiel 2

39 g 2-Methyl-2-cyanhexansäure (Racemat) und 81,1 g (-)Chinin werden in 250 ml Tetrahydrofuran (THF) zum Sieden erhitzt. Nach dem Abkühlen auf Raumtemperatur wird die Lösung in 1 1 Hexan gegeben, das auf -60 °C gekühlt ist. Anschließend rührt man das Gemisch über Nacht bei -20 °C und saugt dann das ausgefallene Salz ab. Das Salz wird aus einer Mischung THF/Hexan (3/7) mehrmals umkristallisiert. Dann löst man das Salz in 10%iger Salzsäure und extrahiert die optisch aktive 2-Methyl-2-cyanhexansäure mit Methyl-tert-butyl-Ether (MTB-Ether). Der MTB-Ether-Extrakt wird mehrmals mit verd. HCI und mit Wasser gewaschen und dann mit Na₂S0₄ getrocknet. Nach dem Abziehen des Lösungsmittels erhält man optisch aktive 2-Methyl-2-cyanhexan- säure.
[a]ö = + 5,5_{°} (0,11 g in 10 ml CHCI₃).

Die so erhaltene Säure wird wie in Beispiel 1 beschrieben mit p-(p-Oxtyloxyphenyl)-phenol verestert. Man erhält optisch aktiven 2-Methyl-2-cyanhexansäure-p-(p-octyloxyphenyl)-phenylester, Fp. 72°.
Analog werden hergestellt:
2-Methyl-2-cyanhexansäure-p-(p-dodecyloxyphenyl)-phenylester
   2-Methyl-2-cyanhexansäure-p-(p-undecyloxyphenyl)-phenylester
   2-Methyl-2-cyanhexansäure-p-(p-decyloxyphenyl)-phenylester
   2-Methyl-2-cyanhexansäure-p-(p-nonyloxyphenyl)-phenylester
   2-Methyl-2-cyanhexansäure-p-(p-heptyloxyphenyl)-phenyl-ester
   2-Methyl-2-cyanhexansäure-p-(p-hexyloxyphenyl)-phenyl-ester, Fp. 50°
   2-Methyl-2-cyanhexansäure-p-(p-pentyloxyphenyl)-phenylester.
   2-Methyl-2-cyanhexansäure-[trans-4-(4-hexyloxybiphenyl-4'-yl)-cyclohexylmethyl]ester
   2-Methyl-2-cyanhexansäure-[trans-4-(4-heptyloxybiphenyl-4'-yl)-cyclohexylmethyl]ester
   2-Methyl-2-cyanhexansäure-[trans-4-(4-octyloxybiphenyl-4'-yl)-cyclohexylmethyl]ester
   2-Methyl-2-cyanhexansäure-[trans-4-(4-nonyloxybiphenyl-4'-yl)-cyclohexylmethyl]ester
   2-Methyl-2-cyanhexansäure-[trans-4-(4-decyloxybiphenyl-4'-yl)-cyclohexylmethyl]ester
2-Methyl-2-cyanhexansäure-[4-(p-hexyloxyphenyl)-biphenyl-4'-yl]ester
   2-Methyl-2-cyanhexansäure-[4-(p-heptyloxyphenyl)-biphenyl-4'-yl]ester
   2-Methyl-2-cyanhexansäure-[4-(p-octyloxyphenyl)-biphenyl-4'-yl]ester
   2-Methyl-2-cyanhexansäure-[4-(p-nonyloxyphenyl)-biphenyl-4'-yl]ester
   2-Methyl-2-cyanhexansäure-[4-(p-decyloxyphenyl)-biphenyl-4'-yl]ester
2-Methyl-2-cyanhexansäure-[p-2-{(p-hexyloxyphenyl)-pyrimidin-2-yl}-phenyl]ester
   2-Methyl-2-cyanhexansäure-[p-2-{(p-heptyloxyphenyl)-pyrimidin-2-yl}-phenyl]ester
   2-Methyl-2-cyanhexansäure-[p-2-((p-octyloxyphenyl)-pyrimidin-2-yl)-phenyl]ester
   2-Methyl-2-cyanhexansäure-[p-2-((p-nonyloxyphenyl)-pyrimidin-2-yl)-phenyl]ester
   2-Methyl-2-cyanhexansäure-[p-2-((p-decyloxyphenyl)-pyrimidin-2-yl)-phenyl]ester
2-Methyl-2-cyanhexansäure-[p-5-{(p-hexyloxyphenyl)-pyrimidin-5-yl}-phenyl]ester
   2-Methyl-2-cyanhexansäure-[p-5-{(p-heptyloxyphenyl)-pyrimidin-5-yl}-phenyl]ester
   2-Methyl-2-cyanhexansäure-[p-5-{(p-octyloxyphenyl)-pyrimidin-5-yl}-phenyl]ester
   2-Methyl-2-cyanhexansäure-[p-5-{(p-nonyloxyphenyl)-pyrimidin-5-yl}-phenyl]ester
   2-Methyl-2-cyanhexansäure-[p-5-{(p-decyloxyphenyl)-pyrimidin-5-yl}-phenyl]ester
2-Methyl-2-cyanhexansäure-[p-5-{(p-hexylphenyl)-pyrimidin-5-yl}-phenyl]ester
   2-Methyl-2-cyanhexansäure-[p-5-{(p-heptylphenyl)-pyrimidin-5-yl}-phenyl]ester
   2-Methyl-2-cyanhexansäure-[p-5-{(p-octylphenyl)-pyrimidin-5-yl}-phenyl]ester
   2-Methyl-2-cyanhexansäure-[p-5-{(p-nonylphenyl)-pyrimidin-5-yl}-phenyl]ester
   2-Methyl-2-cyanhexansäure-[p-5-{(p-decylphenyl)-pyrimidin-5-yl}-phenyl]ester
2-Methyl-2-cyanhexansäure-[p-2-{(p-hexylphenyl)-pyrimidin-2-yl}-phenyl]ester
   2-Methyl-2-cyanhexansäure-[p-2-{(p-heptylphenyl)-pyrimidin-2-yl}-phenyl]ester
   2-Methyl-2-cyanhexansäure-[p-2-{(p-octylphenyl)-pyrimidin-2-yl}-phenyl]ester
   2-Methyl-2-cyanhexansäure-[p-2-{(p-nonylphenyl)-pyrimidin-2-yl}-phenyl]ester
   2-Methyl-2-cyanhexansäure-[p-2-{(p-decylphenyl)-pyrimidin-2-yl}-phenyl]ester

### Beispiel 3

Zu 120 g Phosphoroxidtrichlorid werden unter Rühren 225 g optisch aktives 2-Methyl-2-[5-(p-heptyloxy- phenyl)-pyrimidin-2-yl]octansäureamid bei Raumtemperatur gegeben. Das Reaktionsgemisch wird langsam auf 120° erhitzt und nach 2 Stunden auf Raumtemperatur abgekühlt. Dann gibt man 800 ml Methylenchlorid und vorsichtig 500 ml Wasser zu. Nach üblicherAufarbeitung der organischen Phase erhält man optisch aktives 5-(p-Heptyloxyphenyl)-2-(2-cyan-2-octyl)-pyrimidin.

### Beispiel 4

Zu einer Suspension von 3,26 g 4'-Octyloxybiphenyl-4-carbonsäure, 1,50 g (-)2-Fluor-3-hydroxy-2-methyl- propionsäureethylester (T. Kitazume et al., J.Org.Chem. 51, 1003 (1986)) und einer katalytischen Menge 4-N,N'-Dimethylaminopyridin (DMAP) in 20 ml CH₂Cl₂ werden bei 0 °C bis 5 °C unter Kühlung 2,27 g Dicyclohexylcarbodiimid (DCC) in 10 ml Methylenchlorid gegeben. Man läßt das Reaktionsgemisch unter Erwärmung auf Raumtemperatur über Nacht rühren und saugt dann den Niederschlag ab. Die Methylenchloridphase wird mit verdünnter HCI gewaschen und wie üblich aufgearbeitet. Man erhält optisch aktiven 4'-Octyloxybiphenyl-4-carbonsäure-(2-ethoxycarbonyl-2-fluorpropyl)ester.

### Beispiel 5

Zu einer Suspension von 2,98 g 4'-Octyloxybiphenyl-4-ol, 1,2 g (S)-2-Fluor-2-methylbuttersäure (T. Kitazume et al., J.Org.Chem. Q1, 1003 (1986)) und einer katalytischen Menge DMAP in 20 ml Methylenchlorid werden bei 0 °C bis 5 °C unter Kühlung 2,27 g DCC in 10 ml Methylenchlorid gegeben. Man läßt das Reaktionsgemisch 12 Stunden unter Erwärmung auf Raumtemperatur rühren und saugt dann den Niederschlag ab. Die org. Phase wird wie üblich aufgearbeitet. Man erhält optisch aktiven 4-(2-Fluor-2-methylbutter- säure)-4'-octyloxybiphenylester.
Analog werden hergestellt:
4-(2-Fluor-2-methylbuttersäure)-4'-dodecyloxybiphenylester
   4-(2-Fluor-2-methylbuttersäure)-4'-undecyloxybiphenylester
   4-(2-Fluor-2-methylbuttersäure)-4'-decyloxybiphenylester
   4-(2-Fluor-2-methylbuttersäure)-4'-nonyloxybiphenylester
   4-(2-Fluor-2-methylbuttersäure)-4'-heptyloxybiphenylester
   4-(2-Fluor-2-methylbuttersäure)-4'-hexyloxybiphenylester
   4-(2-Fluor-2-methylbuttersäure)-4'-pentyloxybiphenylester.

### Beispiel 6

Unter Feuchtigkeitsausschluß werden bei -10 ° 29,3 g optisch aktive 2-( trans-4-Heptylcyclohexylmethyl)-2-methylcyanessigsäure [erhältlich aus Cyanessigsäureethylester durch Knoevenagel-Kondensation mit 4- trans-Heptylcyclohexancarbaldehyd, Hydrierung, Alkylierung mit Methyljodid, Verseifung und Racematspaltung], 29,8 g 4'-Octyloxy-4-hydroxybiphenyl und 1,3 g 4-N,N'-Dimethylaminopyridin in 200 ml Dichlormethan vorgelegt und mit 23 g Dicyclohexylcarbodiimid in 30 ml Methylenchlorid versetzt. Nach 12 Stunden Rühren bei Raumtemperatur und üblicher Aufarbeitung erhält man optisch aktiven 2-(trans-4-Heptylcyclohexylmethyl)-2-methylcyanessigsäure-4'-octyloxybiphenyl-4-ylester.

Analog erhält man mit den in Beispiel 1 verwendeten Phenolen bzw. Alkoholen die entsprechenden Ester, z.θ
2-(trans-4-Heptylcyclohexylmethyl)-2-methylcyanessigsäure-4'-butyloxybiphenyl-4-ylester
   2-(trans-4-Heptylcyclohexylmethyl)-2-methylcyanessigsäure-4'-pentyloxybiphenyl-4-ylester
   2-(trans-4-Heptylcyclohexylmethyl)-2-methylcyanessigsäure-4'-hexyloxybiphenyl-4-ylester
   2-(trans-4-Heptylcyclohexylmethyl)-2-methylcyanessigsäure-4'-heptyloxybiphenyl-4-ylester
   2-(trans-4-Heptylcyclohexylmethyl)-2-methylcyanessigsäure-4'-nonyloxybiphenyl-4-ylester
   2-(trans-4-Heptylcyclohexylmethyl)-2-methylcyanessigsäure-4'-decyloxybiphenyl-4-ylester
   2-(trans-4-Heptylcyclohexylmethyl)-2-methylcyanessigsäure-4'-undecyloxybipheny1-4-ylester
   2-(trans-4-Heptylcyclohexylmethyl)-2-methylcyanessigsäure-4'-dodecyloxybipheny1-4-ylester
2-(trans-4-Heptylcyclohexylmethyl)-2-methylcyanessigsäure-p-butyloxyphenylester
   2-(trans-4-Heptylcyclohexylmethyl)-2-methylcyanessigsäure-p-pentyloxyphenylester
   2-(trans-4-Heptylcyclohexylmethyl)-2-methylcyanessigsäure-p-hexyloxyphenylester
   2-(trans-4-Heptylcyclohexylmethyl)-2-methylcyanessigsäure-p-heptyloxyphenylester
   2-(trans-4-Heptylcyclohexylmethyl)-2-methylcyanessigsäure-p-octyloxyphenylester
   2-(trans-4-Heptylcyclohexylmethyl)-2-methylcyanessigsäure-p-nonyloxyphenylester
   2-(trans-4-Heptylcyclohexylmethyl)-2-methylcyanessigsäure-p-decyloxyphenylester
   2-(trans-4-Heptylcyclohexylmethyl)-2-methylcyanessigsäure-p-undecyloxyphenylester
   2-(trans-4-Heptylcyclohexylmethyl)-2-methylcyanessigsäure-p-dodecyloxyphenylester

### Beispiel 7

Unter Feuchtigkeitsausschluß werden 6,28 g optisch aktive 3-(4-Octyloxy-4'-biphenylyl)-2-methyl-2-cy- anpropionsäure [herstellbar aus 4-Octyloxy-4'-biphenylcarbaldehyd durch Knoevenagelreaktion mit Cyanessigsäureethylester unter hydrierenden Bedingungen (analog Org.Synth. III 385), Alkylierung mit Methyljodid und Lithiumdiisopropylamid, Verseifung der Säure und Racematspaltung mit optisch aktiver Base], 0,52 g Methanol und 0,2 g DMAP in 40 ml Methylenchlorid vorgelegt. Das Gemisch wird auf -5 °C bis 0 °C abgekühlt. Dann gibt man 3,6 g DCC in 15 ml Methylenchlorid zu. Unter Erwärmung auf Raumtemperaturwird der Ansatz über Nacht gerührt, dann wird der ausgefallene Dicyclohexylharnstoff abgesaugt und gründlich mit Lösungsmittel ausgewaschen. Das Filtrat wird wie üblich aufgearbeitet. Man erhält optisch aktiven 3-(4'-Octyloxy-4-biphenylyl)-2-methyl-2-cyanpropionsäuremethylester.
Analog werden hergestellt
3-(4'-Dodecyloxy-4-biphenyl-2-methyl-2-cyanpropionsäuremethylester
   3-(4'-Undecyloxy-4-biphenyl-2-methyl-2-cyanpropionsäuremethylester
   3-(4'-Decyloxy-4-biphenyl-2-methyl-2-cyanpropionsäuremethylester
   3-(4'-Nonyloxy-4-biphenyl-2-methyl-2-cyanpropionsäuremethylester
   3-(4'-Heptyloxy-4-biphenyl-2-methyl-2-cyanpropionsäuremethylester
   3-(4'-Hexyloxy-4-biphenyl-2-methyl-2-cyanpropionsäuremethylester
   3-(4'-Pentyloxy-4-biphenyl-2-methyl-2-cyanpropionsäuremethylester
3-(4'-Dodecyloxy-4-biphenyl-2-methyl-2-cyanpropionsäureethylester
   3-(4'-Undecyloxy-4-biphenyl-2-methyl-2-cyanpropionsäureethylester
   3-(4'-Decyloxy-4-biphenyl-2-methyl-2-cyanpropionsäureethylester
   3-(4'-Nonyloxy-4-biphenyl-2-methyl-2-cyanpropionsäureethylester
   3-(4'-Heptyloxy-4-biphenyl-2-methyl-2-cyanpropionsäureethylester
   3-(4'-Hexyloxy-4-biphenyl-2-methyl-2-cyanpropionsäureethylester
   3-(4'-Pentyloxy-4-biphenyl-2-methyl-2-cyanpropionsäureethylester
3-(4'-Dodecyloxy-4-biphenyl-2-methyl-2-cyanpropionsäurepropylester
   3-(4'-Undecyloxy-4-biphenyl-2-methyl-2-cyanpropionsäurepropylester
   3-(4'-Decyloxy-4-biphenyl-2-methyl-2-cyanpropionsäurepropylester
   3-(4'-Nonyloxy-4-biphenyl-2-methyl-2-cyanpropionsäurepropylester
   3-(4'-Heptyloxy-4-biphenyl-2-methyl-2-cyanpropionsäurepropylester
   3-(4'-Hexyloxy-4-biphenyl-2-methyl-2-cyanpropionsäurepropylester
   3-(4'-Pentyloxy-4-biphenyl-2-methyl-2-cyanpropionsäurepropylester
3-(4'-Dodecyloxy-4-biphenyl-2-methyl-2-cyanpropionsäureisopropylester
   3-(4'-Undecyloxy-4-biphenyl-2-methyl-2-cyanpropionsäureisopropylester
   3-(4'-Decyloxy-4-biphenyl-2-methyl-2-cyanpropionsäureisopropylester
   3-(4'-Nonyloxy-4-biphenyl-2-methyl-2-cyanpropionsäureisopropylester
   3-(4'-Heptyloxy-4-biphenyl-2-methyl-2-cyanpropionsäureisopropylester
   3-(4'-Hexyloxy-4-biphenyl-2-methyl-2-cyanpropionsäureisopropylester
   3-(4'-Pentyloxy-4-biphenyl-2-methyl-2-cyanpropionsäureisopropylester
3-(4'-Dodecyloxy-4-biphenyl-2-methyl-2-cyanpropionsäurebutylester
   3-(4'-Undecyloxy-4-biphenyl-2-methyl-2-cyanpropionsäurebutylester
   3-(4'-Decyloxy-4-biphenyl-2-methyl-2-cyanpropionsäurebutylester
   3-(4'-Nonyloxy-4-biphenyl-2-methyl-2-cyanpropionsäurebutylester
   3-(4'-Heptyloxy-4-biphenyl-2-methyl-2-cyanpropionsäurebutylester
   3-(4'-Hexyloxy-4-biphenyl-2-methyl-2-cyanpropionsäurebutylester
   3-(4'-Pentyloxy-4-biphenyl-2-methyl-2-cyanpropionsäurebutylester
3-(4'-Dodecyloxy-4-biphenyl-2-methyl-2-cyanpropionsäurepentylester
   3-(4'-Undecyloxy-4-biphenyl-2-methyl-2-cyanpropionsäurepentylester
   3-(4'-Decyloxy-4-biphenyl-2-methyl-2-cyanpropionsäurepentylester
   3-(4'-Nonyloxy-4-biphenyl-2-methyl-2-cyanpropionsäurepentylester
   3-(4'-Heptyloxy-4-biphenyl-2-methyl-2-cyanpropionsäurepentylester
   3-(4'-Hexyloxy-4-biphenyl-2-methyl-2-cyanpropionsäurepentylester
   3-(4'-Pentyloxy-4-biphenyl-2-methyl-2-cyanpropionsäurepentylester
3-[trans-4-(p-Dodecyloxyphenyl)-cyclohexyl]-2-methyl-2-cyanpropionsäuremethylester
   3-[trans-4-(p-Undecyloxyphenyl)-cyclohexyl]-2-methyl-2-cyanpropionsäuremethylester
   3-[trans-4-(p-Decyloxyphenyl)-cyclohexyl]-2-methyl-2-cyanpropionsäuremethylester
   3-[trans-4-(p-Nonyloxyphenyl)-cyclohexyl]-2-methyl-2-cyanpropionsäuremethylester
   3-[trans-4-(p-Heptyloxyphenyl)-cyclohexyl]-2-methyl-2-cyan propionsäuremethylester
   3-[trans-4-(p-Hexyloxyphenyl)-cyclohexyl]-2-methyl-2-cyanpropionsäuremethylester
   3-[trans-4-(p-Pentyloxyphenyl)-cyclohexyl]-2-methyl-2-cyan propionsäuremethylester
3-[trans-4-(4'-Dodecyloxybiphenyl-4-yl)-cyclohexyl]-2-methyl-2-cyanpropionsäuremethylester
   3-[trans-4-(4'-Undecyloxybiphenyl-4-yl)-cyclohexyl]-2-methyl-2-cyanpropionsäuremethylester
   3-[trans-4-(4'-Decyloxybiphenyl-4-yl)-cyclohexyl]-2-methyl-2-cyanpropionsäuremethylester
   3-[trans-4-(4'-Nonyloxybiphenyl-4-yl)-cyclohexyl]-2-methyl-2-cyanpropionsäuremethylester
   3-[trans-4-(4'-Heptyloxybiphenyl-4-yl)-cyclohexyl]-2-methyl-2-cyanpropionsäuremethylester
   3-[trans-4-(4'-Hexyloxybiphenyl-4-yl)-cyclohexyl]-2-methyl-2-cyanpropionsäuremethylester
   3-[trans-4-(4'-Pentyloxybiphenyl-4-yl)-cyclohexyl]-2-methyl-2-cyanpropionsäuremethylester

### Beispiel 8

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyrimidin,
20 % 2-p-Nonyloxyphenyl-5-nonylpyrimidin,
5 % 2-p-Octyloxyphenyl-5-nonylpyrimidin,
10 % r-1-cyan-cis-4-(4'-heptylbiphenyl-4-yl)-1-hexylcyclohexan,
10 % r-1-cyan-cis-4-(4'-nonyloxybiphenyl-4-yl)-1-octylcyclohexan,
20 % r-1-cyan-cis-4-(4'-octyloxybiphenyl-4-yl)-1-octylcyclohexan,
7 % optisch aktives r-1-cyan-cis-4-(4'-heptyloxybiphenyl-4-yl)-1-(2-methylbutyl)-cyclohexan und
10 % optisch aktiven 2-Methyl-2-ethylcyanessigsäure-p-(5-heptylpyrimidin-2-yl)-phenylester zeigt Sc^{*}/S_{A} 58°, Sy/Ch 63°, Ch/I 78° und eine spontane Polarisation von 17 nC/cm².

### Beispiel 9

Eine achirale Basismischung bestehend aus
3,3 % 2-p-Hexyloxyphenyl-5-heptylpyrimidin,
3,3 % 2-p-Heptyloxyphenyl-5-heptylpyrimidin,
3,3 % 2-p-Octyloxyphenyl-5-heptylpyrimidin,
3,3 % 2-p-Nonyloxyphenyl-5-heptylpyrimidin,
7,8 % 2-p-Hexyloxyphenyl-5-nonylpyrimidin,
25,6 % 2-p-Nonyloxyphenyl-5-nonylpyrimidin,
31,2 % r-1-Cyan-cis-4-(4'-octyloxybiphenyl-4-yl)-1-butylcyclohexan,
15,6 % r-1-Cyan-cis-4-(4'-heptylbiphenyl-4-yl)-1-hexylcyclohexan, und
6,6 % r-1-Cyan-cis-4-(trans-4-pentylcyclohexyl)-1-(trans-4-pentylcyclohexyl)-cyclohexan

wird mit 10 % optisch aktivem 2-Methyl-2-cyanhexansäure-p-(p-octyloxyphenyl)-phenylester (Beispiel 2) versetzt. Die erhaltene chirale getiltete smektische Phase zeigt S*_{C}/S_{A} 66°, S_{A}/Ch 79° CH/I 88° und jeweils bei 20 °C eine Tiltwinkel von 27°, eine spontane Polarisation von 24 nC/cm² und eine Schaltzeit von 69 µs (15 V/µm).

### Beispiel 10

Die achirale Basismischung aus Beispiel 9 wird mit 10 % 2-Methyl-2-cyanhexansäure-p-(p-hexyloxyphenyl)-phenylester (Beispiel 2) versetzt. Die erhaltene chirale getiltete smektische Phase zeigt S^{*}_{c}/S_{A} 61 °, S_{A}/Ch 76°, Ch/I 86°, und jeweils bei 20 °C einen Tiltwinkel von 29°, eine spontane Polarisation von 25 nC/cm² und eine Schaltzeit von 66 µs (15 V/µm).

## Patentansprüche

1. Chirale oder achirale Ringverbindungen der Formel 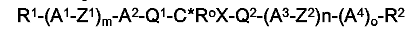 worin
R¹ und R² jeweils unabhängig voneinander eine Alkyloder Perfluoralkyl-Gruppe mit jeweils 1-12 C-Atomen, worin auch eine oder zwei nicht benachbarte CH₂- bzw. CF₂-Gruppen durch O-Atome und/oder -CO-Gruppen und/oder -CO-O-Gruppen und/oder -CH=CH-Gruppen und/oder -CHHalogen- und/oder - CHCN-Gruppen und/oder -O-CO-CHHalogen- und/oder -CO-O-CHCN-Gruppen ersetzt sein können,
eine der Gruppen R¹ und R² auch H, F, Cl, Br oder CN, R¹ auch -Q¹-C*R°X-Q²-R², wobei Q¹, R^{o}, X, Q² und R² die angegebenen Bedeutungen haben,
A¹, A², A3 und A⁴jeweils unabhängig voneinander unsubstituiertes oder durch ein oder zwei F- und/oder CI-Atome und/oder CH₃-Gruppen und/oder CN-Gruppen substituiertes 1,4-Phenylen, worin auch eine oderzwei CH-Gruppen durch N ersetzt sein können, 1,4-Cyclohexylen, worin auch ein oder zwei nicht benachbarte CH₂-Gruppen durch O-Atome und/oder S-Atome ersetzt sein können, Piperidin-1,4-diyl, 1,4-Bicyclo(2,2,2)octylen-, 1,3,4-Thiadiazol-2,5-diyl, Naphthalin -2,6-diyl-, Decahydronaphthalin-2,6-diyl-oder 1,2,3,4-Tetrahydronaphthalin-2,6-diyl-,
Z¹ und Z² jeweils -CO-O-, -O-CO-, -CH₂CH₂-, -OCH₂-, -CH₂0-, -C≡C- oder eine Einfachbindung, X Halogen, CN oder CH₃,
R^{o} eine von X (und im Falle n= o= 0 auch von R²) verschiedene Alkylgruppe mit 1 bis 10 C-Atomen, C^{*} ein mit vier verschiedenen Substituenten verknüpftes Kohlenstoffatom,
m und n jeweils unabhängig voneinander 0, 1, 2 oder 3,
o 0 oder 1,
(m + n + o) 1, 2, 3 oder 4, und
Q¹ und Q² jeweils unabhängig voneinander Alkylen mit 1 bis 4 C-Atomen, worin auch eine CH₂-Gruppe durch -O-, -S-, -CO-, -O-CO-, -CO-O-, -S-CO-, -CO-S-, -CH=CH-COO-, -CH=CH-, -CHHalogen und/oder -CHCN- ersetzt sein kann, oder eine Einfachbindung
bedeuten.

2. Ringverbindungen nach Anspruch 1 der Formeln la1 bis la4 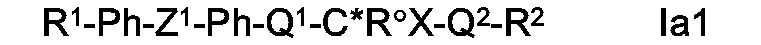 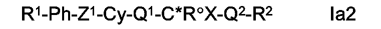 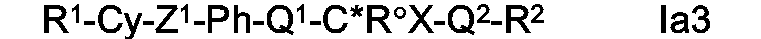 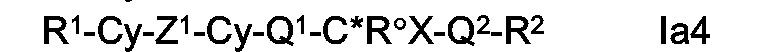 worin Z¹, Q¹, Q² R¹, R², R^{o} und X die angegebene Bedeutung besitzen, und
Ph eine 1,4-Phenylengruppe, worin auch eine oder zwei CH-Gruppen durch N ersetzt sein können, und
Cy eine 1,4-Cyclohexylengruppe, worin auch eine oder zwei nicht benachbarte CH₂-Gruppen durch O-Atome ersetzt sein können,
bedeuten.

3. Ringverbindungen nach Anspruch 1 der Formel Ic1 bis Ic8 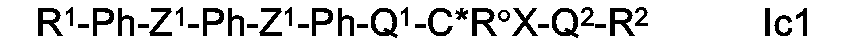 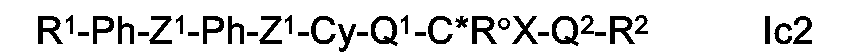 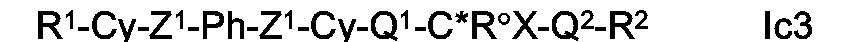 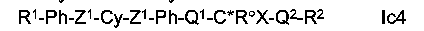 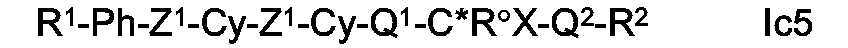 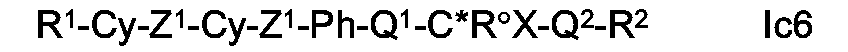 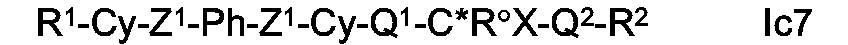 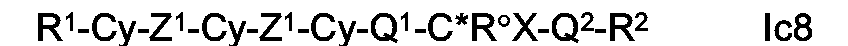 worin R¹, Z¹, Q¹, Q², R^{o}, X und R² die in Anspruch 1 gegebene Bedeutung besitzen und Ph und Cy die in Anspruch 2 gegebene Bedeutung besitzen.

4. Verbindungen der Formel I' 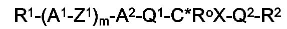 worin m 1 oder 2 bedeutet und R¹, R², A¹, A², Z¹, Q¹, Q², R^{o} und X die angegebene Bedeutung haben, wobei R° eine von X und Q²⁻R² verschiedene Alkylgruppe ist.

5. Verbindungen der Formel nach einem der Ansprüche 1-4, worin -(A¹-Z¹)ₘ-A²- eine Gruppe derfolgenden Formeln 1 bis 38 oder deren Spiegelbild bedeutet:

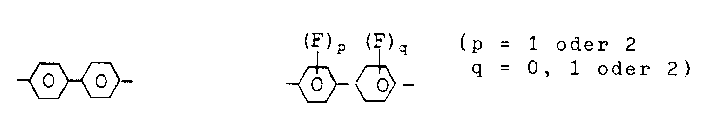

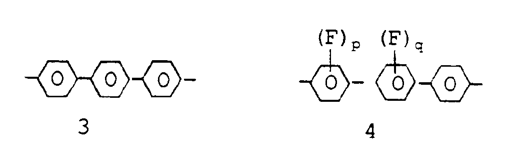

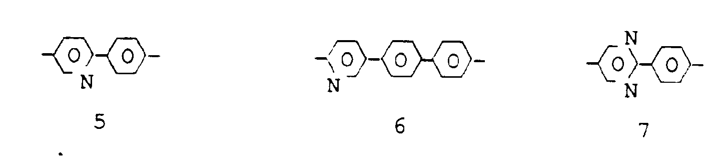

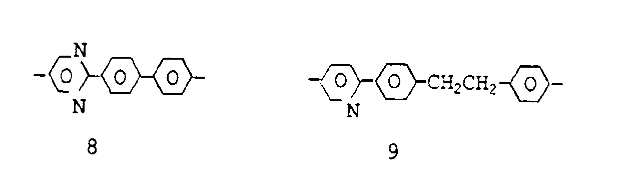

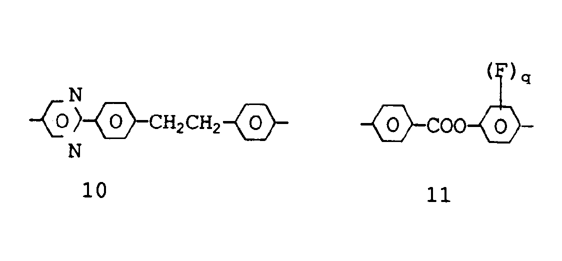

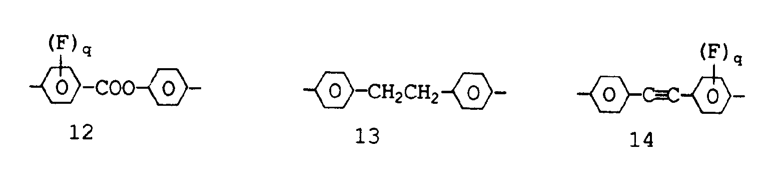

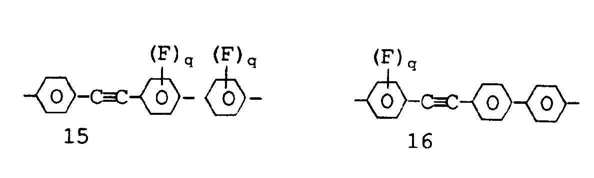

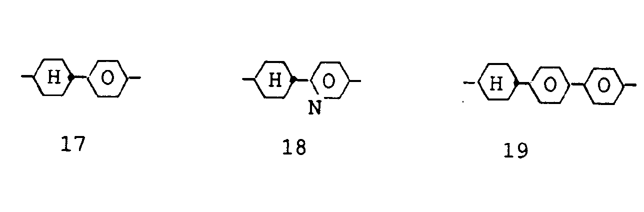

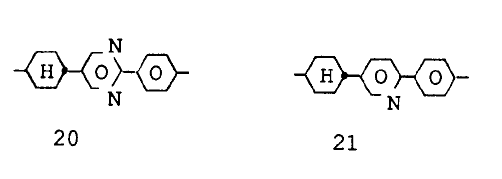

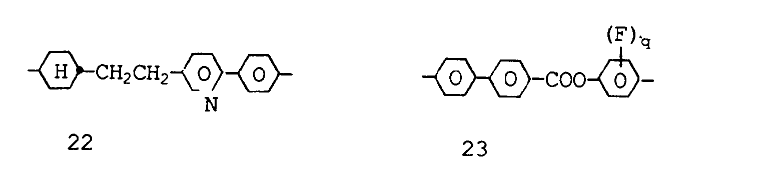

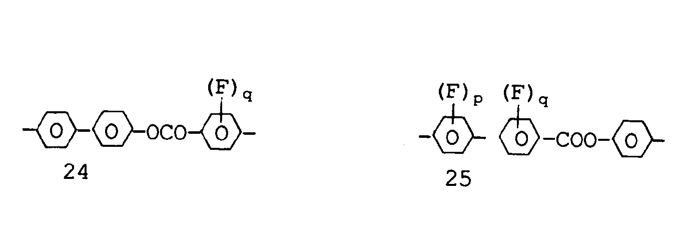

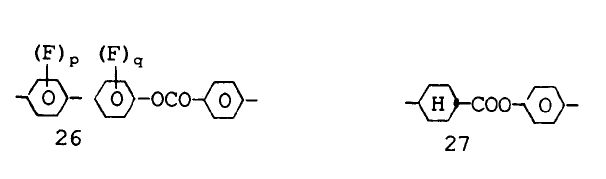

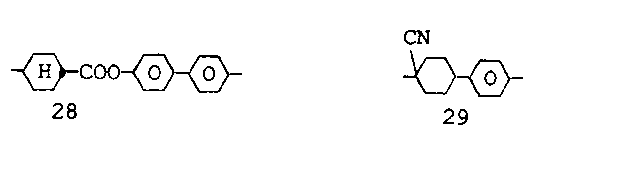

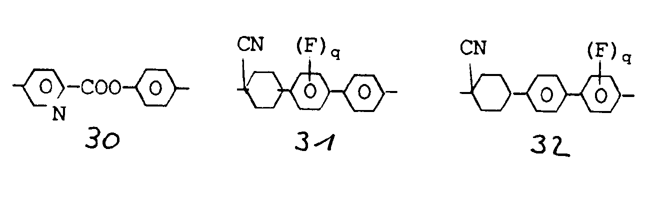

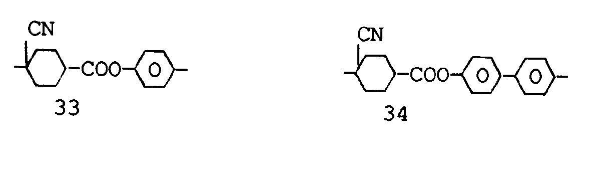

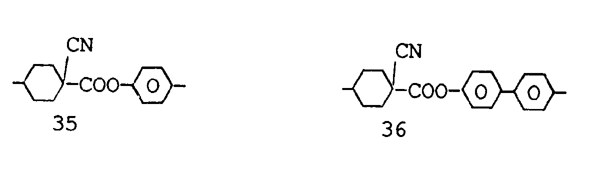

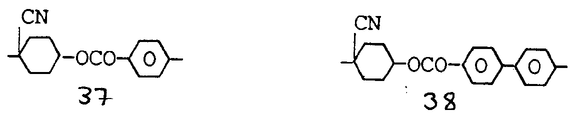

6. Verbindungen nach einem der Ansprüche 1 bis 5 ausgewählt aus den Teilformeln B, C, D, E, F und G: 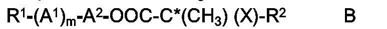 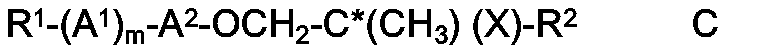 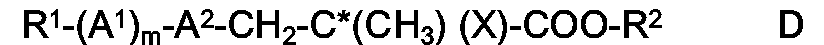 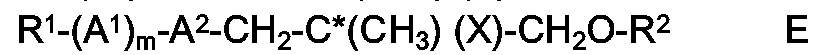 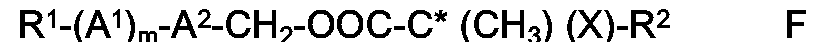 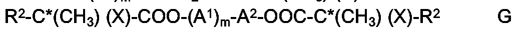 worin R¹, R², A¹, X und A² die bei Formel angegebene Bedeutung haben und m 1 oder 2 ist.

7. Verbindungen nach Anspruch 6, worin -(A¹)ₘ-A²- 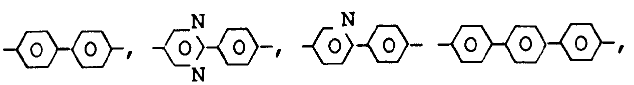 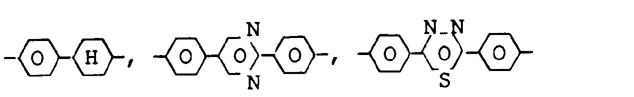 oder 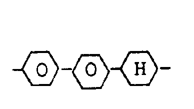 oder ein Spiegelbild dieser Formeln bedeutet.

8. Chirale getiltete smektische flüssigkristalline Phase mit mindestens zwei flüssigkristallinen Komponenten, dadurch gekennzeichnet, daß sie mindestens eine Verbindung der Formel nach Anspruch 1 enthält.

9. Phase nach Anspruch 8, dadurch gekennzeichnet, daß sie mindestens eine der Verbindungen der Teilformeln Va bis Vp: 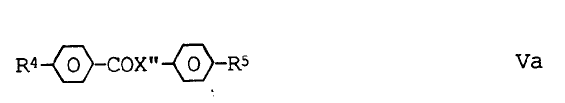 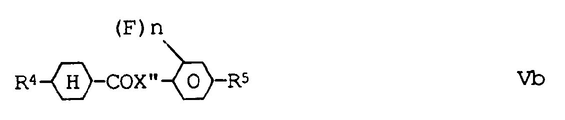 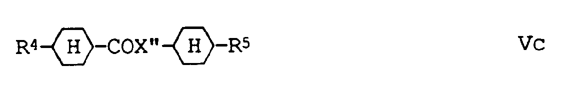 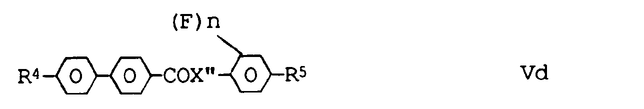 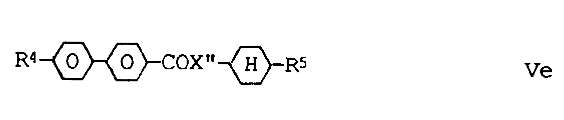 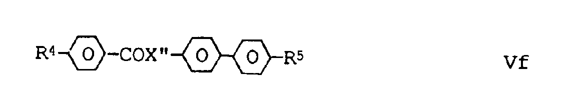 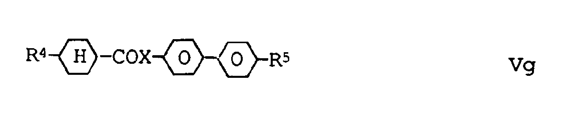 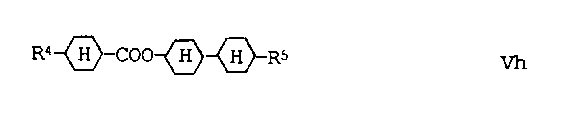 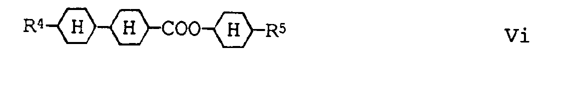 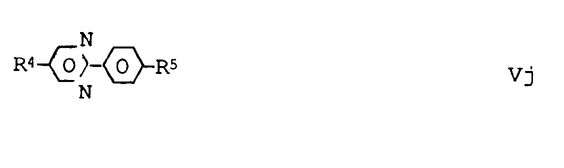 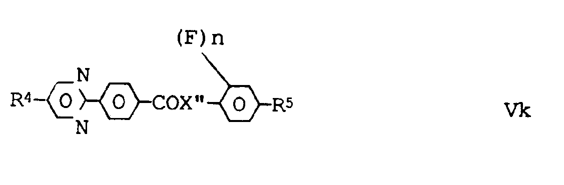 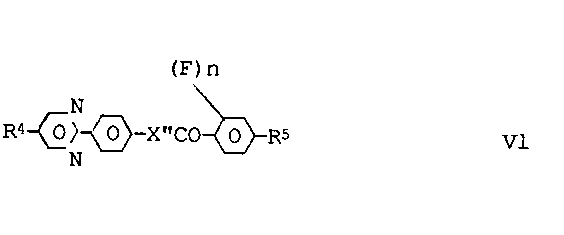 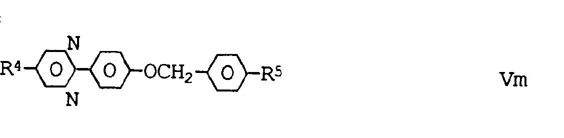 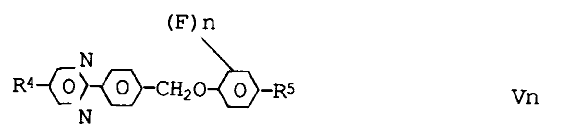 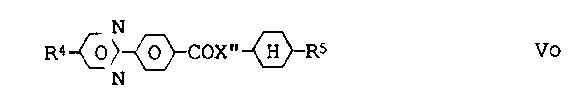 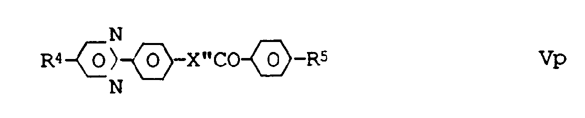 worin R⁴ und R⁵ jeweils geradkettiges oder verzweigtes Alkyl, Alkoxy, Alkanoyloxy oder Alkoxycarbonyl mit jeweils 3 bis 12 C-Atomen, X" O oder S und n 0 oder 1 bedeuten.

10. Verwendung derVerbindungen der Formel nach Anspruch 1 als Komponenten flüssigkristalliner Phasen.

11. Elektrooptisches Anzeigeelement, dadurch gekennzeichnet, daß es als Dielektrikum eine Phase nach Anspruch 8 enthält.

## Claims

1. Chiral or achiral ring compounds of the formula I 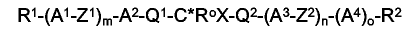 in which
R¹ and R² are each, independently of one another, an alkyl or perfluoroalkyl group which has, in each case, 1-12 C atoms and in which it is also possible for one or two non-adjacent CH₂ or CF₂ groups to be replaced by O atoms and/or -CO- groups and/or -CO-O- groups and/or -CH=CH- groups and/or - CHhalogen- and/or -CHCN- groups and/or -O-CO-CHhalogen- and/or -CO-O-CHCN- groups,
one of the groups R¹ and R² is also H, F, Cl, Br or CN, R¹ is also -Q¹-C*R^{o}X-Q²-R², where Q¹, R^{o}, X, Q² and R² have the indicated meanings,
A¹, A², A3 and A4 are each, independently of one another, 1, 4-phenylene which is unsubstituted or substituted by one or two F and/or CI atoms and/or CH₃ groups and/or CN groups and in which it is also possible for one or two CH groups to be replaced by N, 1,4-cyclohexylene in which it is also possible for one or two non-adjacent CH₂ groups to be replaced by O atoms and/or S atoms, piperidine-1,4 -diyl, 1,4- bicyclo(2,2,2)-octylene (sic), 1,3,4-thiadiazole-2,5-diyl, naphthalene-2,6-diyl-, decahydronaphthalene-2,6-diyl- or 1,2,3,4-tetrahydronaphthalene-2,6-diyl-,
Z¹ and Z² are each -CO-O-, -O-CO-, -CH₂CH₂-, -OCH₂-, -CH₂0-, -C≡C- or a single bond,
X is halogen, CN or CH₃,
R^{o} is an alkyl group which has 1 to 10 C atoms and differs from X (as well as from R² in the case where n = o = 0 ),
C^{*} is a carbon atom which is linked to four different substituents,
m and n are each, independently of one another, 0, 1, 2 or 3
o is 0 or 1,
(m + n + o) is 1, 2, 3 or 4, and
Q¹ and Q² are each, independently of one another, alkylene which has 1 to 4 C atoms and in which it is also possible for a CH₂ group to be replaced by -O-, -S-, -CO-, -O-CO-, -CO-O-, -S-CO-, -CO-S-, - CH=CH-COO-, -CH=CH-, -CHhalogen and/or -CHCN-, or are each a single bond.

2. Ring compounds according to Claim 1 of the formulae la1 to la4 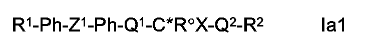 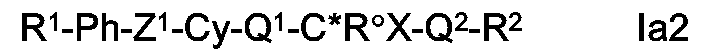 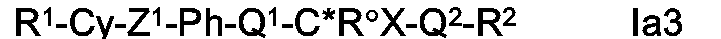 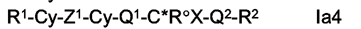 in which Z¹, Q^{l}, Q² R¹, R², R^{o} and X have the stated meaning, and
Ph is a 1,4-phenylene group in which it is also possible for one or two CH groups to be replaced by N, and
Cy is a 1,4-cyclohexylene group in which it is also possible for one or two non-adjacent CH₂ groups to be replaced by O atoms.

3. Ring compounds according to Claim 1 of the formula Ic1 to Ic8 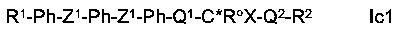 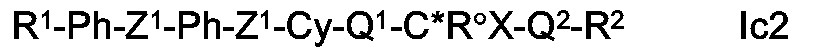 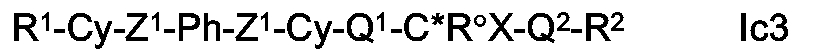 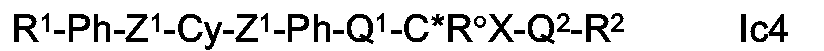 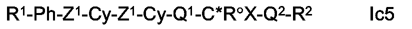 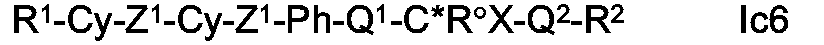 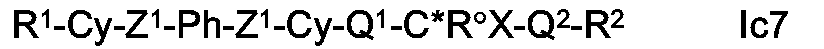 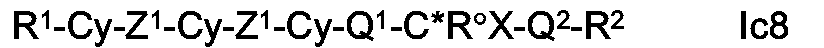 in which R¹, Z¹, Q¹, Q¹, R^{o}, X and R² have the meaning given in Claim 1, and Ph and Cy have the meaning given in Claim 2.

4. Compounds of the formula I' 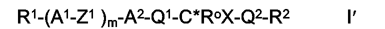 in which m is 1 or 2, and R¹, R², A¹, A², Z¹, Q¹, Q², R^{o} and X have the stated meaning, where R^{o} is an alkyl group which differs from X and Q²⁻R².

5. Compounds of the formula I according to any of Claims 1-4, in which -(A¹-Z¹)ₘ-A²- is a group of the following formulae 1 to 38 or the mirror image thereof:

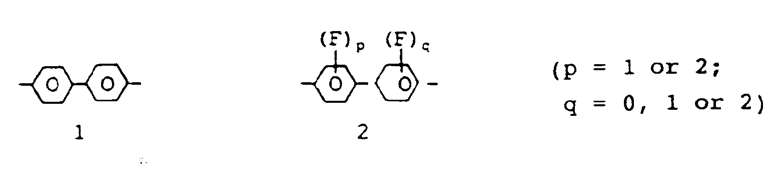

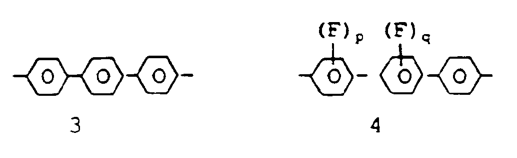

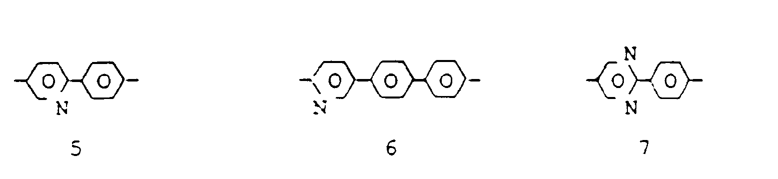

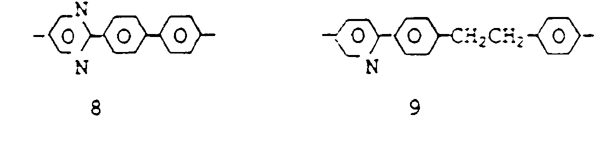

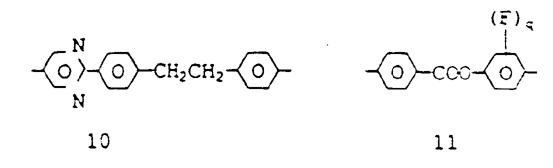

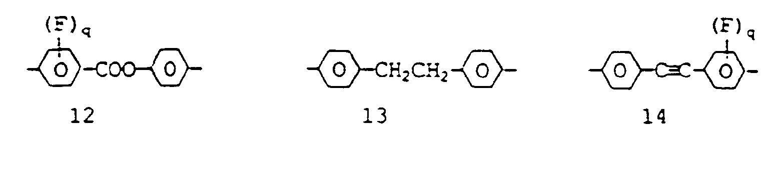

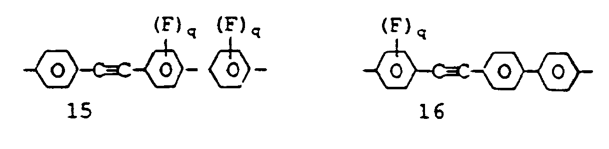

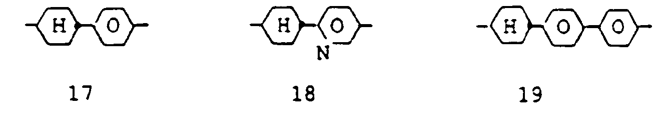

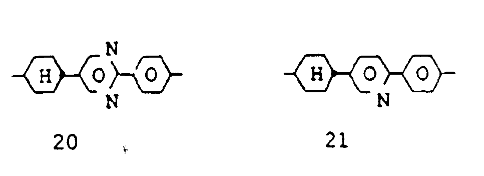

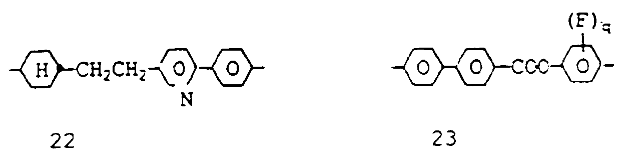

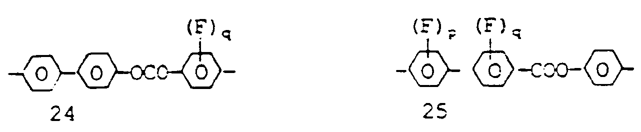

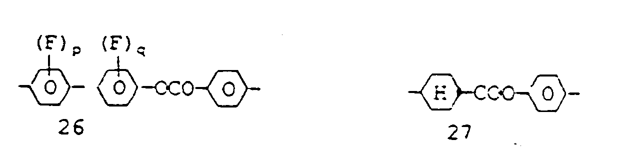

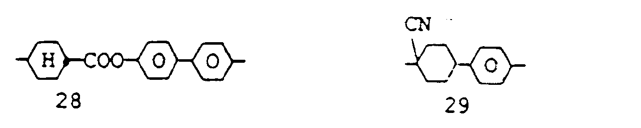

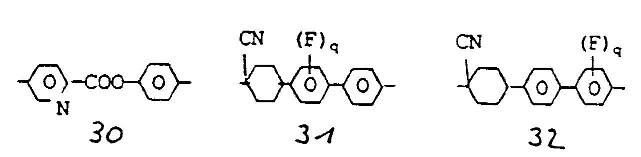

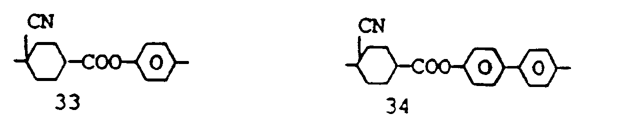

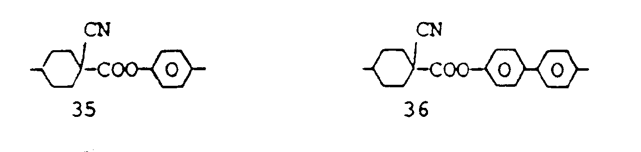

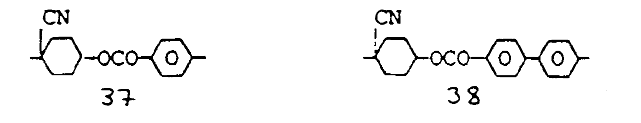

6. Compounds according to any of Claims 1 to 5 selected from the part-formulae B, C, D, E, F and G: 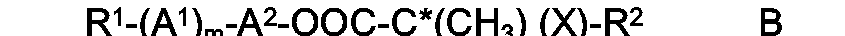 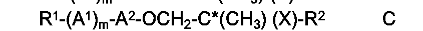 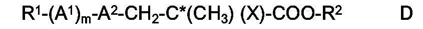 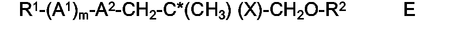 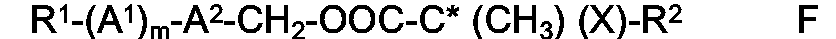 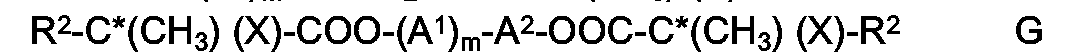 in which R¹, R², A¹, X and A² have the meaning indicated for formula I, and m is 1 or 2.

7. Compounds according to Claim 6, in which -(A¹)ₘ-A²- is 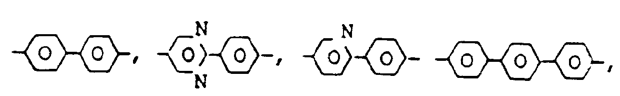 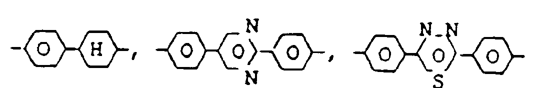 or 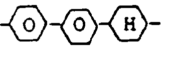 or a mirror image of these formulae.

8. Chiral tilted smectic liquid crystalline phase having at least two liquid crystalline components, characterised in that it contains at least one compound of the formula I according to Claim 1.

9. Phase according to Claim 8, characterised in that it (lacuna) at least one of the compounds of the part-formulae Va to Vp: 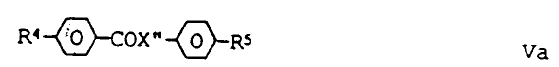 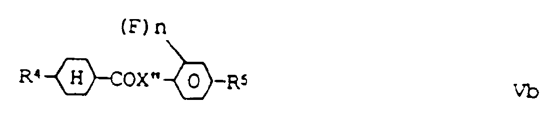 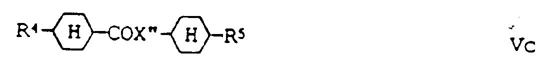 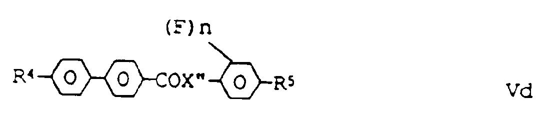 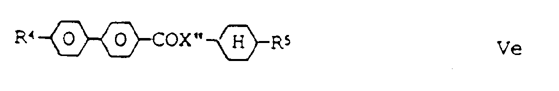 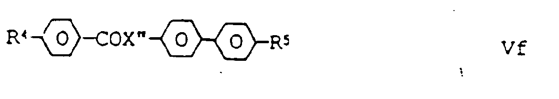 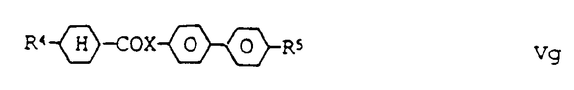 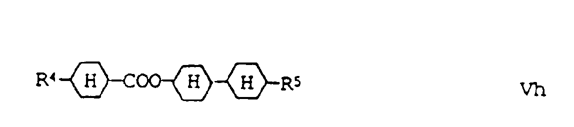 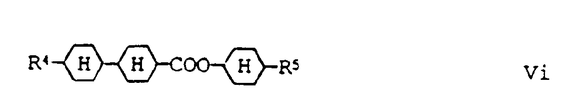 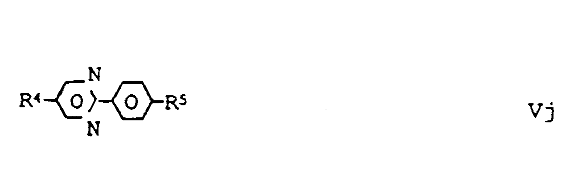 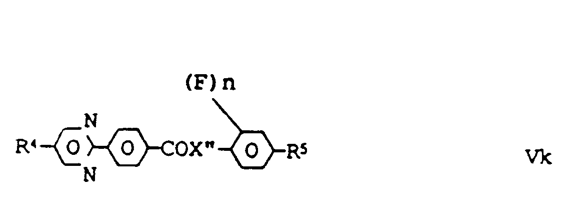 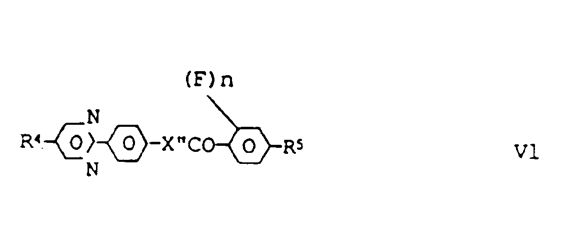 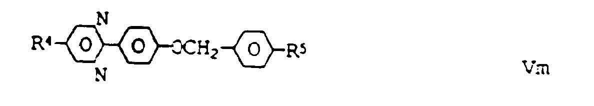 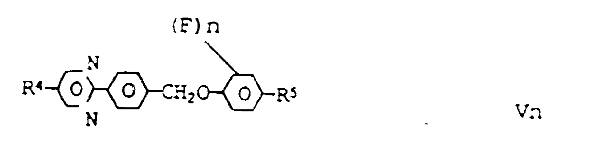 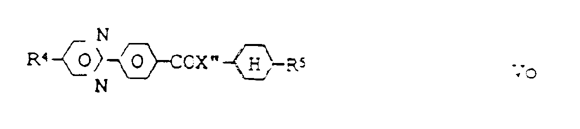 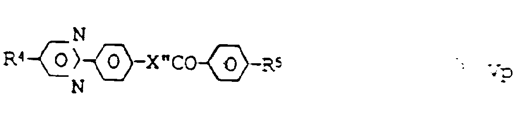 in which R⁴ and R⁵ are each straight-chain or branched alkyl, alkoxy, alkanoyloxy or alkoxycarbonyl having, in each case, 3 to 12 C atoms, X" is O or S, and n is 0 or 1.

10. Use of the compounds of the formula I according to Claim 1 as components of liquid crystalline phases.

11. Electrooptical display element, characterised in that it contains as dielectric a phase according to Claim 8.

## Revendications

1.- Composés cycliques chiraux ou achiraux de formule 1 : 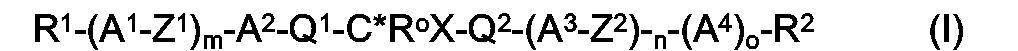 dans laquelle :
R¹ et R² indépendamment l'un de l'autre représentent un groupe alkyle ou perfluoroalkyle avec 1-12 atome de C, dans lequel aussi un ou deux groupes CH₂- ou CF₂ peuvent être remplacés par des atomes 0 et/ou des groupes -CO- et/ou des groupes -CO-O- et/ou. des groupes -CH=CH- et/ou -CH-halogène et/ou des groupes -CHCN et/ou des groupes -O-CO-CH-halogène et/ou des groupes -CO-O-CHCN, l'un des groupes R¹ et R² peut être aussi H, F, CI, Brou CN, R¹ peut être aussi -Q¹-C*R^{o}X-Q²-R², dans laquelle Q¹, R^{o}, X, Q² et R² ont les significations données,
A¹, A², A³ et A⁴ représentent indépendamment l'un de l'autre un 1,4-phénylène non substitué ou substitué par un ou deux atomes de F et/ou de CI et/ou des croupes CH₃ et/ou des groupes CN, dans lequel on peut remplacer un ou deux groupes CH par N, un 1,4-cyclohexylène dans lequel aussi un ou deux groupes CH₂ non contigus peuvent être remplacés par des atomes de O ou de atomes de S, pipéridin-1,4-diyle, 1,4-bicyclo-(2,2,2)-octy- lène-, 1,3,4-thiadiazol-2,5-diyle, naphtalène-2,6-diyle, décahydronaphtalène-2,6-diyle ou 1,2,3,4-tétrahydro- naphtalène-2,6-diyle,
Z¹ et Z² représentent chacun -CO-O-, -O-CO-, -CH₂CH₂-, -OCH₂-, -CH₂0-, -C≡C- ou une liaison simple,
X représente un halogène, CN ou CH₃,
R^{o} un groupe alkyle différent de X (et aussi de R² dans le cas où n = o = 0) avec 1 à 10 atomes de C,
C^{*} un atome de carbone relié à quatre substituants différents,
m et n sont indépendamment l'un de l'autre 0, 1, 2 ou 3,
o est 0 ou 1,
(m+n+o) est 1, 2, 3 ou 4 et
Q¹ et Q² représentent indépendamment l'un de l'autre des alkyles avec 1 à 4 atomes de C, dans lesquels aussi un groupe CH₂ peut être remplacé par -O-, -S-, -CO-, -O-CO-, -CO-O-, -S-CO-, -CO-S-, -CH=CH-COO-, - CH=CH-, -CH-halogène, et/ou -CH-CN-, ou une liaison simple.

2.- Composés cycliques selon la revendication 1 de formule la1 à la4 : 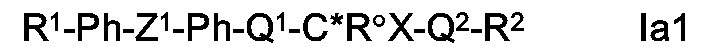 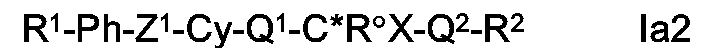 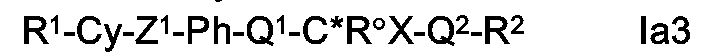 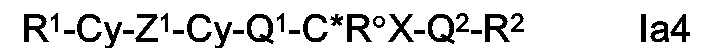 dans lesquelles Z¹, Q¹, Q² R¹, R², R² et X ont la signification donnée et
Ph est un groupe 1,4-phénylène, dans lequel on peut remplacer aussi un ou deux groupes CH par N,
Cy est un groupe 1,4-cyclohexylène, dans lequel on peut remplacer aussi un ou deux groupes CH₂ non contigus jar des atomes de O.

3.- Composés cycliques selon la revendication 1 de formule Ic1 à Ic8 : 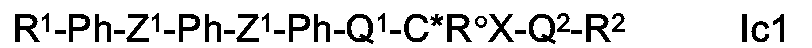 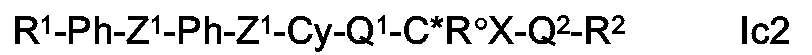 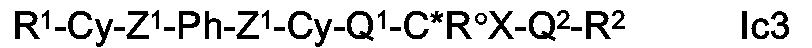 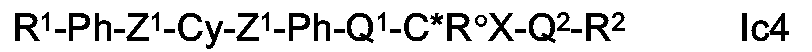 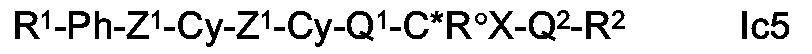 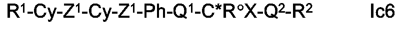 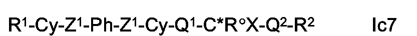 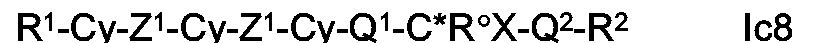 dans lesquelles R¹, Z¹, Q¹, Q², R^{o}, X et R² ont la signification donnée à la revendication 1 et Ph et Cy ont la signification indiquée à la revendication 2.

4.- Composés de formule l' : 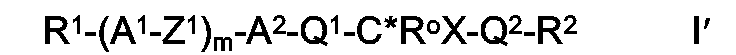 dans laquelle m est 1 ou 2 et R¹, R2, A¹, A², Z¹, Q¹, Q², R^{o} et X ont la signification indiquée, dans laquelle R° est un groupe alkyle différent de X et de Q²⁻R².

5.- Composés de formule I selon l'une des revendications 1-4, dans lesquels -(A¹-Z¹)ₘ-A²- représente un groupe des formules suivantes 1 à 38 ou leur image dans un miroir : 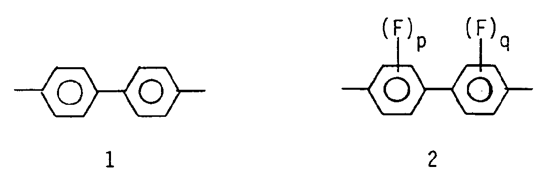
p = 1 ou 2
q=0,1ou2

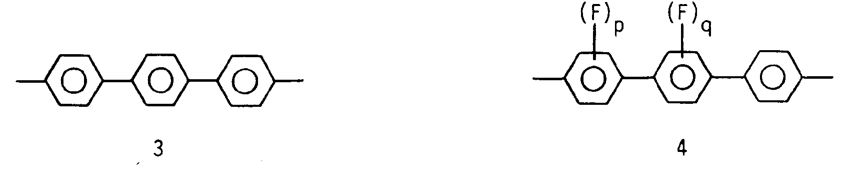

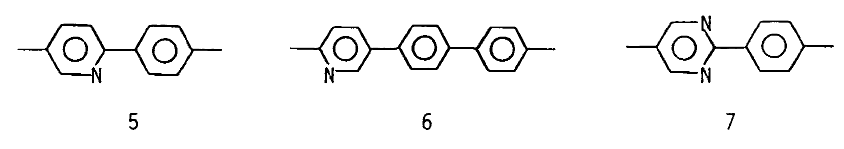

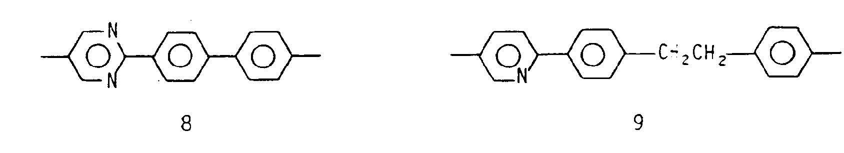

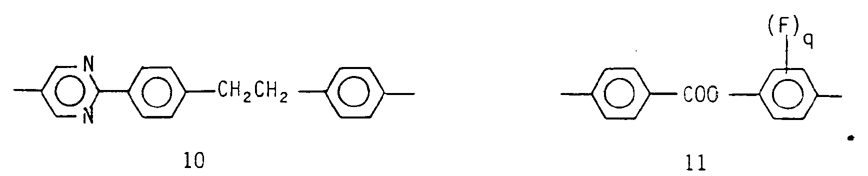

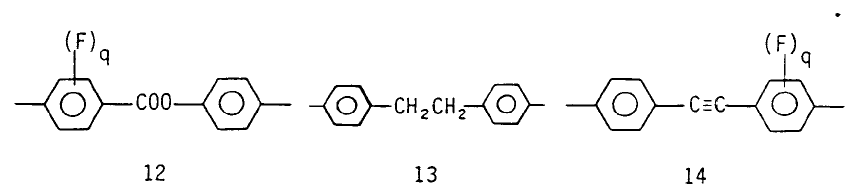

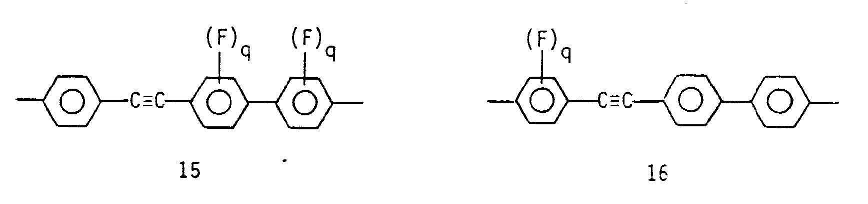

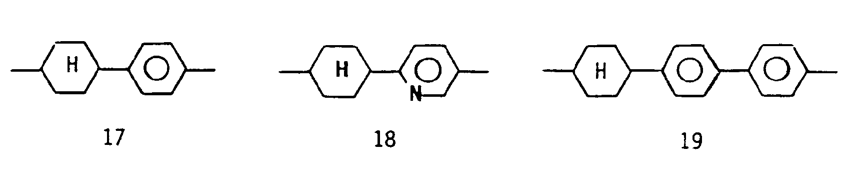

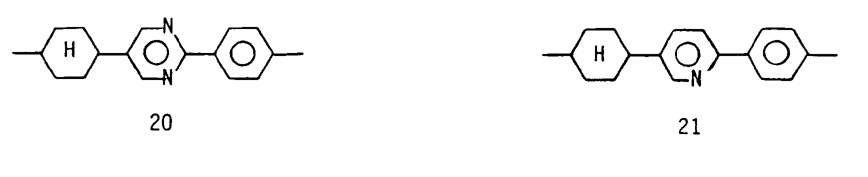

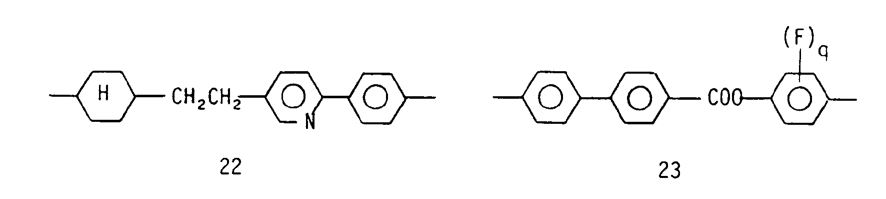

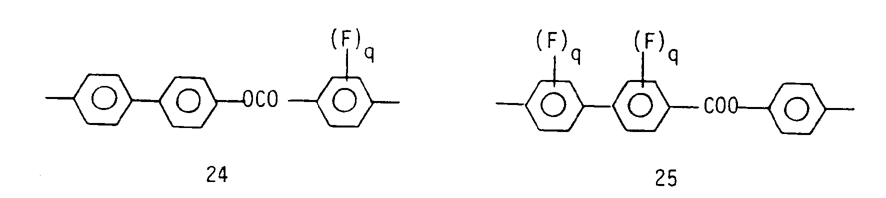

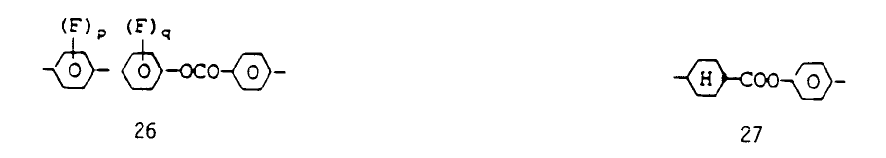

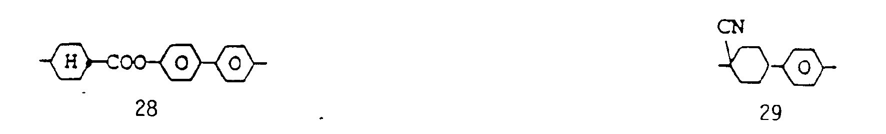

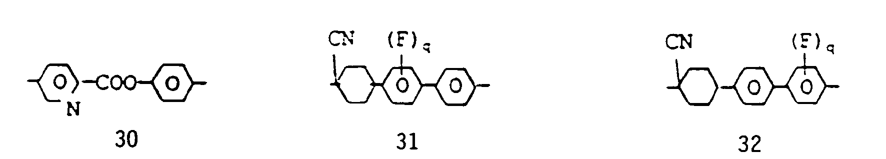

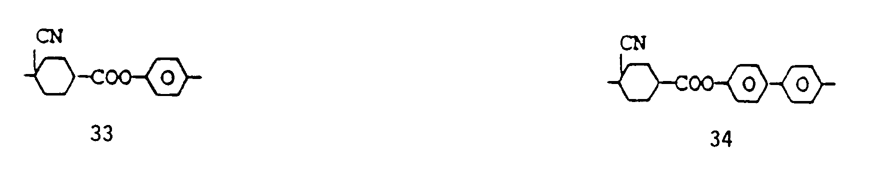

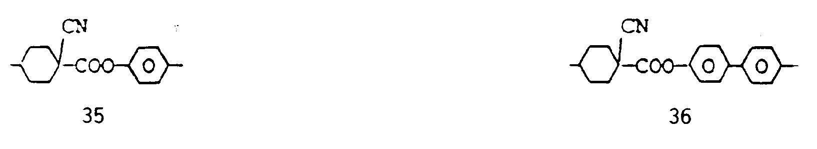

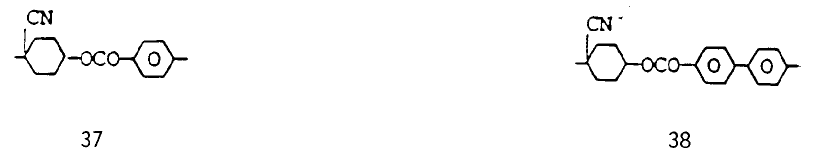

6.- Composés selon l'une des revendications 1 à 5 choisis parmi les formules partielles B, C, D, E, F et G : 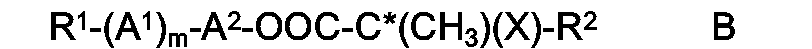 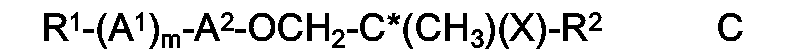 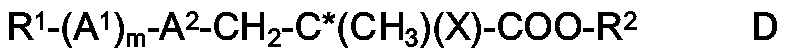 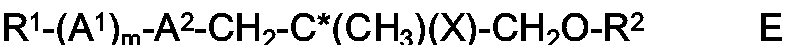 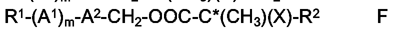 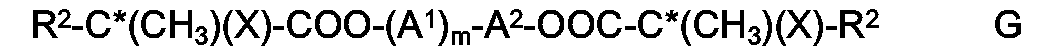 dans lesquelles R¹, R², A¹, X et A² ont les significations données et m est 1 ou 2.

7.- Composés selon la revendication 6, dans lesquels -(A¹)ₘ-A² représente : 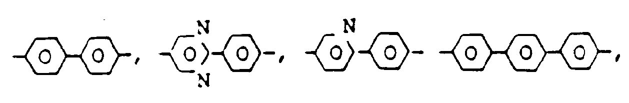 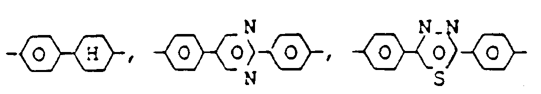 ou 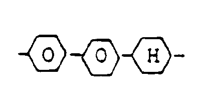 ou l'image dans un miroir de ces formules.

8.- Phase de cristaux liquides smectique chirale ayant au moins deux composants cristaux liquide, caractérisée en ce qu'elle contient au moins un composé de formule I selon la revendication 1.

9.- Phase selon la revendication 8, caractérisée en ce qu'elle contient au moins l'un des composés de formules partielles Va à Vp : 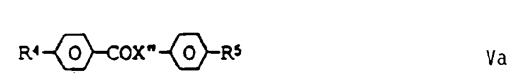 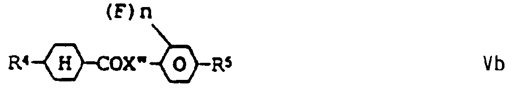 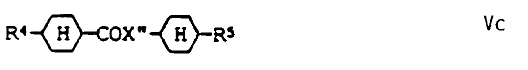 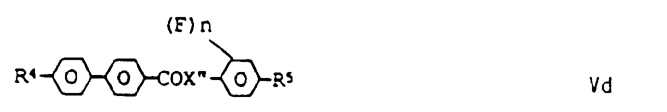 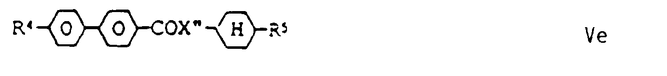 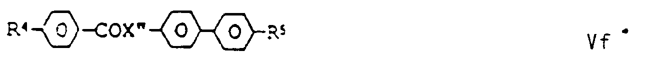 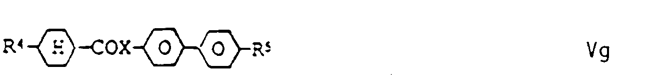 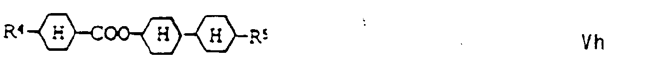 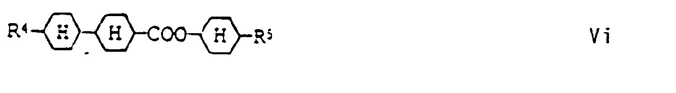 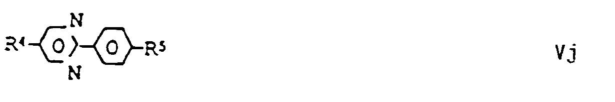 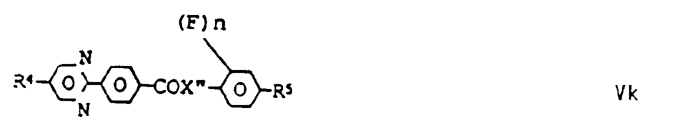 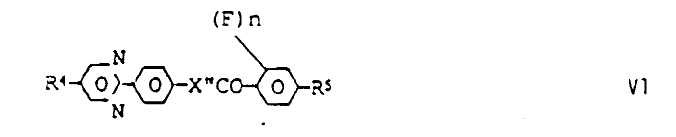 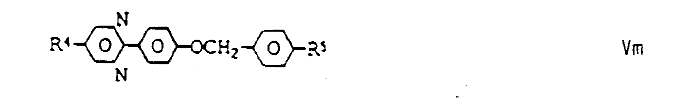 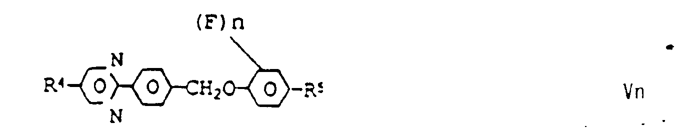 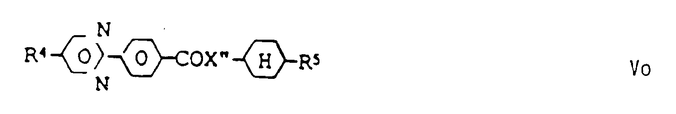 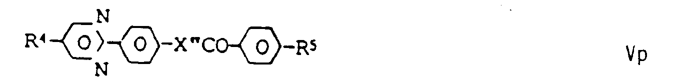 dans lesquelles R⁴ et R⁵ représentent chacun un alkyle, alcoxy, alcanoyloxy ou alcoxycarbonyle linéaire ou ramifié, avec chaque fois de 3 à 12 atomes de C, X" est O ou S et n est 0 ou 1.

10.- Utilisation de composés de formule 1 selon la revendication 1 comme composants de phases à cristaux liquides.

11.- Elément d'affichage électro-optique, caractérisé en ce qu'il contient comme diélectrique une phase selon la revendication 8.
